# EUROPÄISCHE PATENTSCHRIFT

(11) **EP 3 016 984 B1**
(45) Veröffentlichungstag und Bekanntmachung des Hinweises auf die Patenterteilung: **26.12.2018**
(21) Anmeldenummer: 14734819.7
(22) Anmeldetag: 02.07.2014
(51) Int. Cl.: C08F 120/06, C08F 283/06, C11D 3/37, C08F 2/24, C08F 2/44, C08L 33/02

(54) **VERWENDUNG EINER GELFÖRMIGEN POLYMERZUSAMMENSETZUNG, ERHÄLTLICH DURCH POLYMERISATION EINES SÄUREGRUPPENHALTIGEN MONOMERS IN GEGENWART EINER POLYETHERVERBINDUNG IN FORMULIERUNGEN FÜR DIE MASCHINELLE GESCHIRRREINIGUNG**
USE OF A GEL-LIKE POLYMER COMPOSITION WHICH CAN BE OBTAINED BY POLYMERIZING AN ACID GROUP-CONTAINING MONOMER IN THE PRESENCE OF A POLYETHER COMPOUND IN FORMULATIONS FOR AUTOMATIC DISHWASHING
UTILISATION D'UNE COMPOSITION POLYMÈRE GÉLIFORME, OBTENUE PAR POLYMÉRISATION D'UN MONOMÈRE CONTENANT DES GROUPES ACIDES EN PRÉSENCE D'UN COMPOSÉ POLYÉTHER DANS DES FORMULATIONS POUR LE LAVAGE DE VAISSELLE EN MACHINE

(30) Priorität: 03.07.2013 EP 13174938
(43) Veröffentlichungstag der Anmeldung: 11.05.2016
(73) Patentinhaber: BASF SE, 67056 Ludwigshafen am Rhein (DE)
(72) Erfinder: WEBER, Heike, 68239 Mannheim (DE); FUCHS, Yannick, 69469 Weinheim (DE); WITTELER, Helmut, 67157 Wachenheim (DE); BÖHN, Roland, 67133 Maxdorf (DE); RIEGER, Ralph, 67063 Ludwigshafen (DE)
(86) Internationale Anmeldenummer: PCT/EP2014/064083
(87) Internationale Veröffentlichungsnummer: WO 2015/000969

(56) Entgegenhaltungen:
- EP-A1- 0 519 603
- WO-A1-2005/012378
- WO-A1-2012/069440
- US-A- 5 384 061
- LEV BROMBERG: "Novel Family of Thermogelling Materials via C-C Bonding between Poly(acrylic acid) and Poly(ethylene oxide)- b -poly(propylene oxide)- b -poly(ethylene oxide)", THE JOURNAL OF PHYSICAL CHEMISTRY B, Bd. 102, Nr. 11, 20. Februar 1998 (1998-02-20), Seiten 1956-1963, XP055113924, ISSN: 1520-6106, DOI: 10.1021/jp9803687

## Beschreibung

### HINTERGRUND DER ERFINDUNG

Die vorliegende Erfindung betrifft die Verwendung einer gelförmigen Polymerzusammensetzung, zu deren Herstellung man eine α,β-ethylenisch ungesättigte Säure einer radikalischen Polymerisation in Gegenwart wenigstens einer Polyetherkomponente unterzieht, in Formulierungen für die maschinelle Geschirrreinigung (automatic dish washing, ADW).

### STAND DER TECHNIK

Maschinelle Geschirreinigungsverfahren im häuslichen und im gewerblichen Bereich umfassen mehrere aufeinanderfolgende Schritte, wobei der erste das mechanische Abtragen lose anhaftender Speisereste umfasst, der zweite den eigentlichen Reinigungsvorgang mit Hilfe einer Geschirrspülmaschine darstellt und der dritte in der Regel aus einem Klarspülschritt besteht, an den sich das Trocknen des gereinigten Geschirrs anschließt. Diese Vorgänge werden mehr oder weniger automatisiert durchgeführt, wobei als zentrale Einheit eine Geschirrspülmaschine zum Einsatz kommt, in der zumindest der Reinigungsschritt und in der Regel auch der anschließende Klarspülschritt und/oder der Trocknungsschritt durchgeführt werden.

In Geschirrspülmaschinen für den häuslichen Bereich erfolgt die Reinigung des verschmutzten Geschirrs in der Regel in einer einzelnen Kammer, wobei die zuvor genannten Behandlungsschritte programmgesteuert nacheinander ablaufen. Frischwasser gelangt über die Enthärtungseinheit zum Pumpensumpf und wird mittels bewegter Sprüharme über dem zu spülenden Gut versprüht. Abgespülte wasserunlösliche Stoffe werden am Pumpensumpf herausgefiltert. Beim zweiten Spülgang wird ein in der Regel alkalisches Reinigungsmittel dem Spülwasser zugesetzt, auf die eingestellte Temperatur erhitzt und auf dem zu spülenden Gut verteilt. Beim letzten Spülgang wird der Behandlungsflüssigkeit ein Klarspülmittel zugesetzt, das die Oberflächenspannung reduziert, wodurch die Behandlungsflüssigkeit leichter vom Spülgut abläuft. Nach dem letzten Spülgang wird der Inhalt getrocknet. Die im Spülvorgang eingesetzten Komponenten, wie Wasseraufbereiter, Reinigungsmittel, Klarspülmittel, etc., können entweder in Form von Einzelkomponenten oder in Mehrkomponentenformulierungen eingesetzt werden. Solche multifunktionalen Reiniger enthalten Tenside für das Klarspülen und ein Polymer für die Wasserenthärtung. In die Geschirrspülmaschine muss dann nicht separat ein Klarspülmittel und ein Salz für die Wasserenthärtung gegeben werden.

Gewerbliche Geschirrspülmaschinen bestehen im Prinzip aus stationären Bädertanks, aus denen eine im Wesentlichen wässrige Reinigungslösung auf das Geschirr aufgedüst oder -gesprüht wird, das sich auf einem Laufband über diesen Bädern vorbeibewegt, so dass die gebrauchte Lösung wieder in die Bädertanks zurück gelangt. Dabei tritt Wasser in den letzten Bädertank ein, fließt durch Überläufe kaskadenartig durch alle anderen Tanks und verlässt die Maschine durch den Überlauf des ersten Tanks. Das Aufbringen einer in der Regel hochalkalischen Reinigungslösung findet in der Regel mit Hilfe dafür vorgesehener Düsen oder einer speziellen Sprühanlage statt, die normalerweise im mittleren Bereich der Maschine angeordnet ist.

In Formulierungen für die maschinelle Geschirrreinigung werden häufig Polyether oder Tenside zusammen mit Polyacrylsäure eingesetzt, wobei der Polyacrylsäure die Rolle eines Inkrustierungsinhibitors oder Dispergiermittels zukommt. Es besteht das Problem, dass Polyether und Tenside mit Polyacrylsäure in flüssiger Form häufig nicht oder nur bedingt verträglich sind, so dass es beim Mischen zur Phasenseparation oder zu Ausfällungen kommt, was die Einsatzmöglichkeiten wenigstens einer der Komponenten stark einschränkt. Es ist insbesondere bislang nicht gelungen, transparente Gelformulierungen bereitzustellen, die Polyetherole oder Polyethergruppen-haltige Tenside in Kombination mit Säuregruppen-haltigen Polymeren und speziell mit Polyacrylsäure enthalten. Gelförmige Formulierungen werden jedoch vom Verbraucher sowohl aufgrund ihrer anwendungstechnischen Eigenschaften, wie auch aus ästhetischen Gründen bevorzugt.

Für viele aktuelle Anwendungen müssen gelbildende oder filmbildende Polymerzusammensetzungen ein komplexes Anforderungsprofil erfüllen.

Aus der Literatur sind zahlreiche Verfahren zur Herstellung von Gelen aus Polyacrylsäure bekannt. Diese Gele sind zumeist aber nicht in Wasser löslich, da sie auf vernetzter Polyacrylsäure basieren. Andere Gele sind zwar wasserlöslich, basieren aber auf Copolymeren der Acrylsäure mit hydrophoben Monomeren und sind deshalb als Inkrustierungsinhibitoren oder Dispergiermittel weniger leistungsfähig als reine Polyacrylsäure. Weitere Gele basieren auf hochmolekularer Polyacrylsäure, was aber für die Anwendung als Inkrustierungsinhibitor oder Dispergiermittel ebenfalls nicht vorteilhaft ist und die Probleme bei der Verarbeitung noch verstärkt.

In der EP 0 971 997 B1 wird eine flüssige Reinigerformulierung beschrieben, die ein nichtionisches Tensid und ein anionisches Polymer enthält. Das Polymer weist ein Molekulargewicht von mehr als 100 000 g/mol auf. Die Formulierung ist eine ca. 25%ige wässrige Lösung. Gelförmige Formulierungen für die maschinelle Geschirrreinigung sind nicht beschrieben.

Die EP 0 499 068 A1 beschreibt Reaktionsprodukte aus Alkoxilaten und vinylischen Monomeren, wobei diese zumindest teilweise funktionelle Gruppen tragen, die mit den OH-Gruppen der Alkoxilate in einer Kondensation reagieren können. Zur Herstellung dieser Reaktionsprodukte polymerisiert man entweder die vinylischen Monomere in Gegenwart der Alkoxilate und unterzieht das Produkt der Polymerisation anschließend einer Kondensation oder man polymerisiert zuerst die vinylischen Monomere und unterzieht anschließend das Produkt der Polymerisation einer Kondensation mit den Alkoxilaten. Im Falle, dass Acrylsäure als vinylisches Monomer eingesetzt wird, ist das Reaktionsprodukt somit in jedem Fall ein Ester der Polyacrylsäure. In den Ausführungsbeispielen werden als Alkoxilate ausschließlich EO-PO-Blockcopolymere mit hohem PO-Gehalt und Polytetrahydrofuran eingesetzt. Die beschriebenen Polymere dienen als Emulsionsspalter zur Schnellentwässerung von Rohölen. Ein Einsatz in gelförmigen Formulierungen für die maschinelle Geschirrreinigung ist nicht beschrieben.

Ein ähnliches Verfahren wird in der DE 4326772 A1 beschrieben. Hier wird zusätzlich Toluol oder Xylol als Lösungsmittel verwendet. Die Verwendung von aromatischen Lösungsmitteln ist für Produkte, die in Konsumgütern eingesetzt werden sollen, unerwünscht, da die vollständige Entfernung des Lösemittels sehr zeit- und energieaufwendig ist. Die Reaktionsprodukte sind Flüssigkeiten, die als veresterte Polyacrylsäuren beschrieben werden. Esterbildung läuft der Wirkung des Polymers, beispielsweise als Inkrustierungsinhibitor, zuwider und ist nicht erwünscht in Anwendungen, in denen der Einsatz möglichst reiner Polyacrylsäure gefordert ist. Auch in diesem Dokument ist ein Einsatz der Polymere in gelförmigen Formulierungen für die maschinelle Geschirrreinigung nicht beschrieben.

X. Li et al. beschreiben im Journal of Solid State Electrochemistry (2011), 15(6), 1271-1277 und J. H. Wu et al. beschreiben in Advanced Materials, 2007, 19, 4006-4011, Herstellungsverfahren für Polyacrylsäure-Polyethylenglykol-Gele für den Einsatz in Farbstoff-sensitivierten Solarzellen.

In der WO 2008/139151 A1 wird ein Verfahren beschrieben, in dem Polyethylenglycol mit Acrylsäure, Isobornylacrylat und weiteren Komponenten gemischt und durch UV-Belichtung zu einem festen Gel ausgehärtet ist. Aufgrund der Zusammensetzung ist für den Fachmann ersichtlich, dass das Gel nicht wasserlöslich ist. Die Gele dienen als Indikator, dass ein Datenträger, z. B. eine computerlesbare Compactdisc, nicht zuvor benutzt wurde.

In der WO 2010/026178 A2 wird auf Seite 62 und in Beispiel 19 eine Fällungspolymerisation beschrieben, bei der Acrylsäure in Gegenwart eines Tensids polymerisiert wird. Die Handhabung einer großen Menge an organischen Lösungsmitteln bezogen auf relativ wenig Acrylsäure und Tensid ist für dieses Verfahren erforderlich. Außerdem enthält das Reaktionsprodukt eine große Menge an Vinylpyrrolidon und Alkyl-PEG-Methacrylat einpolymerisiert, was das Reaktionsprodukt als Inkrustierungsinhibitor ungeeignet macht. Ein Einsatz der erhaltenen Gele für die maschinelle Geschirrreinigung ist wiederrum nicht beschrieben.

F. E. Bailey et al. beschreiben in Polymer Preprints, American Chemical Society, Division of Polymer Chemistry, 1960, Vol. 1, Issue 2, p. 202-205 und der darin zitierten Literatur die Bildung von molekularen Assoziationskomplexen von Ethylenoxid-Polymeren, die ein sehr hohes Molekulargewicht aufweisen, mit polymeren Säuren, wie Polyacrylsäure, in wässrigen Lösungen. In Abhängigkeit vom eingesetzten Lösungsmittel, dem pH-Wert und der Konzentration der Polymere kommt es zur Ausfällung der Komplexe oder diese bleiben in Lösung. Zur Herstellung fester Gele ist es erforderlich, die Komponenten in Lösungsmitteln auflösen, dann zu mischen und das Lösungsmittel wieder zu entfernen, was mit zusätzlichen Prozessschritten verbunden ist, die ein solches Verfahren für den praktischen Gebrauch unwirtschaftlich machen. Eine Verwendung der Gele für die maschinelle Geschirrreinigung ist nicht offenbart.

Lev Bromberg beschreibt im Journal of Physical Chemistry B (1998), 102, 11, 1956-1963, ein Material mit thermoreversibler Gelbildung, zu dessen Herstellung man Acrylsäure in Gegenwart eines PEO-PPO-PEO-Blockcopolymers polymerisiert. Die Reaktion erfolgt in Abwesenheit externer Lösungsmittel, um einen hohen Anteil an Verzweigung und Vernetzung in den erhaltenen Produkten zu erzielen. Diese sind weder wasserlöslich noch transparent. Als mögliche Einsatzbereiche für diese Polymere werden nur ganz allgemein die Pharmazie und die Nahrungsergänzung genannt (S. 1956, linke Spalte, "Introduction").

WO 2004/099274 beschreibt ein Verfahren zur Herstellung von Polymermischungen durch Polymerisation von Monomeren vom (Meth)acrylsäuretyp in Gegenwart einer Verbindung mit einer Polyalkylenglycol-Struktur. Die erhaltenen Polymermischungen sollen Anteile von Pfropfcopolymeren enthalten, wobei auch nach längerer Lagerung die Polyalkylenglycol-Komponente und die (Meth)acrylsäure-Komponente homogen in der Mischung verbleiben. Die Polymerisation erfolgt zwingend in Gegenwart von Wasser mit einem hohen Wassergehalt in der Vorlage zu Beginn der Reaktion. Die nach dem Verfahren erhaltenen Polymermischungen eignen sich für diverse Detergenzanwendungen, speziell zur Wiederanschmutzverhinderung.

EP 0 639 592 A1 beschreibt Pfropfcopolymere, die durch Polymerisation einer (Meth)acrylsäure-haltigen Polymerzusammensetzung in Gegenwart einer Polyetherverbindung mit mehr als 80 Mol-% Ethylenoxideinheiten erhältlich sind. Die Polymerisation erfolgt im Wesentlichen ohne Lösungsmittel und bei Temperaturen oberhalb von 100 °C. Es wird als kritisch für die Erzielung hoher Pfropfgrade angesehen, dass der Lösungsmittelgehalt des Reaktionsgemischs keinesfalls mehr als 5 Gew.-% beträgt. Die erhaltenen Polymere dienen als Builder für Flüssigwaschmittel oder, gegebenenfalls nach einer Nachvernetzung, als wasserabsorbierende Harze.

WO 2005/012378 beschreibt wässrige Dispersionen von wasserlöslichen Polymeren von anionischen Monomeren und deren Verwendung. Beispiel 4 (Seite 19, Zeilen 14-27) betrifft die Polymerisation von Acrylsäure in Gegenwart von Stabilisatoren, bei denen es sich um Polypropylenglycole handelt. Die Dispersionen werden unter Anderem in Personal care-Produkten sowie in Wasch- und Reinigungsmitteln eingesetzt. Ein Einsatz in Gelform für die maschinelle Geschirrreinigung ist nicht beschrieben.

US5384061 offenbart die Verwendung einer gelförmigen Polymerzusammensetzung in Formulierungen für die maschinelle Geschirrreinigung. Die Zusammensetzung enthält eine Polycarbonsäure, die 0.1 bis 10 Gew % eines Vernetzers mit mindestens zwei α,β-ethylenisch ungesättigte Doppelbindungen enthält.

EP0519603 offenbart auch die Verwendung einer gelförmigen Polymerzusammensetzung in Formulierungen für die maschinelle Geschirrreinigung. Die Zusammensetzung enthalt eine Polyacrylsäure, die in Gegenwart einer Polyetherkomponente polymerisiert wurde. Insbesondere offenbaren die Beispiele Carbopol 614, eine Polyacrylsäure, die mit einem Polyether mit Alkylenoxid Widerholungseinheiten vernetzt wurde.

Der vorliegenden Erfindung liegt die Aufgabe zugrunde, eine gelförmige Polymerzusammensetzung für die Verwendung in Formulierungen für die maschinelle Geschirrreinigung bereit zu stellen, wobei die zuvor beschriebenen Nachteile des Stands der Technik vermieden werden.

Überraschenderweise wurde nun gefunden, dass diese Aufgabe gelöst wird, wenn in Formulierungen für die maschinelle Geschirrreinigung eine Polymerzusammensetzung verwendet wird, zu deren Herstellung eine Monomerzusammensetzung auf Basis wenigstens einer α,β-ethylenisch ungesättigten Säure einer radikalischen Polymerisation in Gegenwart einer Polyetherkomponente unterzogen wird.

### ZUSAMMENFASSUNG DER ERFINDUNG

Ein erster Gegenstand der Erfindung ist die Verwendung einer gelförmigen Polymerzusammensetzung, erhältlich durch ein Verfahren, bei dem man
a) eine Monomerzusammensetzung M) bereitstellt, die aus
   A) wenigstens einer α,β-ethylenisch ungesättigten Säure und
   B) 0 bis 0,1 Gew.-%, bezogen auf das Gesamtgewicht der Monomerzusammensetzung M), vernetzend wirkenden Monomeren, die zwei oder mehr als zwei polymerisierbare α,β-ethylenisch ungesättigte Doppelbindungen pro Molekül aufweisen,
   besteht,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon,
in Formulierungen für die maschinelle Geschirrreinigung.

In einer speziellen Ausführungsform umfasst die Polyetherkomponente PE) wenigstens ein Polyetherol oder einen Mono- oder Di-(C₁-C₂-alkylether) davon oder besteht die Polyetherkomponente PE) aus wenigstens einem Polyetherol oder einem Mono- oder Di-(C₁-C₂-alkylether) davon, das als Alkylenoxideinheiten überwiegend oder ausschließlich Ethylenoxideinheiten eingebaut enthält.

Wenn die Polyetherkomponente PE) ein Polyetherol mit Propylenoxid-Wiederholungseinheiten oder einen Mono- oder Di-(C₁-C₆-alkylether) eines Polyetherols mit Propylenoxid-Wiederholungseinheiten umfasst, beträgt der Anteil dieser Propylenoxid-Wiederholungseinheiten vorzugsweise im Mittel höchstens 18 Einheiten pro Molekül.

Wenn die Polyetherkomponente PE) ein Polyetherol mit Propylenoxid-Wiederholungseinheiten oder einen Mono- oder Di-(C₁-C₆-alkylether) eines Polyetherols mit Propylenoxid-Wiederholungseinheiten umfasst, beträgt der Anteil dieser Propylenoxid-Wiederholungseinheiten vorzugsweise im Mittel höchstens 17 Einheiten pro Molekül, besonders bevorzugt höchstens 15 Einheiten pro Molekül, spezieller höchstens 10 Einheiten pro Molekül.

In einer weiteren speziellen Ausführungsform erfolgt die radikalische Polymerisation in Schritt b) in Gegenwart eines Lösungsmittels LM), das ausgewählt ist unter Wasser, C₁-C₆-Alkanolen, von PE) verschiedenen Polyolen, deren Mono- und Dialkylethern und Mischungen davon. In einer speziellen Ausführung zur Herstellung transparenter Gele ist das Lösungsmittel LM) ausgewählt unter 1,2-Propylenglycol, den isomeren Dipropylenglycolen und Mischungen davon.

In einer weiteren speziellen Ausführungsform unterzieht man das Reaktionsgemisch während der Polymerisation in Schritt b) und die in Schritt b) erhaltene Polymerzusammensetzung keiner Kondensation unter Entfernung eines niedermolekularen Reaktionsprodukts und/oder in Gegenwart eines Kondensationskatalysators.

Ein weiterer Gegenstand der Erfindung ist ein Verfahren zur maschinellen Reinigung von Geschirr, bei dem man das zu reinigende Geschirr mit einer Behandlungslösung in Kontakt bringt, die eine Formulierung für die maschinelle Geschirrreinigung, wie zuvor und im Folgenden definiert, enthält.

### BESCHREIBUNG DER ERFINDUNG

Die erfindungsgemäße Verwendung von gelförmigen Polymerzusammensetzungen in Formulierungen für die maschinelle Geschirrreinigung weist die folgenden Vorteile auf:
- Die erfindungsgemäß verwendeten Polymerzusammensetzungen können in Form klarer Gele bereitgestellt werden. Sie eignen sich für Formulierungen für die maschinelle Geschirrreinigung, die in einer speziellen Ausführung ihrerseits gelförmig sind.
- Die eingesetzten Polymerzusammensetzungen zeichnen sich sowohl durch eine hohe Verträglichkeit zwischen Polyetherkomponente und Polyacrylsäure als auch durch eine hohe Verträglichkeit mit weiteren Tensiden aus.
- Bei der Herstellung der in den erfindungsgemäß verwendeten Formulierungen eingesetzten Polymerzusammensetzungen kann weitgehend oder vollständig auf den Einsatz vernetzender Monomere verzichtet werden. Die so erhaltenen Polymerzusammensetzungen sind vorteilhafterweise wasserlöslich.

Die erfindungsgemäßen Polymerzusammensetzungen liegen unter Normalbedingungen (20 °C) als Gele vor. "Gelförmige Konsistenz" zeigen Formulierungen, die eine höhere Viskosität als eine Flüssigkeit aufweisen und die insbesondere selbsttragend sind, d. h. die eine ihnen verliehene Form ohne formstabilisierende Umhüllung behalten. Im Gegensatz zu festen Formulierungen lassen sich gelförmige Formulierungen jedoch leicht durch Erwärmen und/oder unter Anwendung von Scherkräften deformieren oder in eine fluide Form überführen. Die Viskosität der gelförmigen Polymerzusammensetzungen liegt vorzugsweise bei 20°C in einem Bereich von größer als 600 bis etwa 10 000 000 mPas, besonders bevorzugt von 1000 bis 1 000 000 mPas, insbesondere von 2 000 bis 500 000 mPas. Die Viskosität bei 20°C sowie der Viskositätsverlauf in Abhängigkeit der Temperatur der zu untersuchenden Proben wurden mittels eines Rotationsrheometers (DHR-1 der Firma TA-Instruments mit Peltiersystem, Platte/Platte-Geometrie, Ø 40 mm, h =1 mm) bei Temperaturen von 20 °C bis 80 °C untersucht. Temperatur-Rampe (γ = 1 % mit *M*ₘᵢₙ = 100 µNm). Messtemperatur(en) von 80 °C zu 20 °C und zurück je zwei Läufe (Kühl-/Heizrate 2 K/min). Messzeit je 30 min.

Im Rahmen dieser Anmeldung werden Verbindungen, die von Acrylsäure und Methacrylsäure abgeleitet sein können, teilweise durch Einfügen der Silbe "(meth)" in die von der Acrylsäure abgeleitete Verbindung bezeichnet.

Im Rahmen der vorliegenden Erfindung umfasst der Ausdruck Alkyl geradkettige und verzweigte Alkylgruppen. Dazu zählen vorzugsweise C₁-C₄₀-Alkylgruppen, besonders bevorzugt C₁-C₃₀-Alkylgruppen.

Geeignete kurzkettige Alkylgruppen sind z. B. geradkettige oder verzweigte C₁-C₆-Alkylgruppen, bevorzugt C₁-C₄-Alkylgruppen. Geeignete längerkettige Alkylgruppen sind z. B. geradkettige oder verzweigte C₇-C₄₀-Alkylgruppen, bevorzugt C₈-C₃₀-Alkylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, bzw. um überwiegend lineare Alkenylreste, wie sie auch in natürlichen oder synthetischen Fettsäuren und Fettalkoholen sowie Oxoalkoholen vorkommen, die einfach, zweifach oder mehrfach ungesättigt sein können.

Geeignete Alkylgruppen sind insbesondere Methyl, Ethyl, Propyl, Isopropyl, n-Butyl, 2-Butyl, sec.-Butyl, tert.-Butyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, etc.

Im Rahmen der vorliegenden Erfindung umfasst der Ausdruck Alkenyl geradkettige und verzweigte Alkenylgruppen. Geeignete Alkenylgruppen können eine oder mehrere C-C-Doppelbindungen aufweisen. Dazu zählen vorzugsweise C₂-C₄₀-Alkenylgruppen, besonders bevorzugt C₄-C₃₀-Alkenylgruppen. Geeignete längerkettige C₈-C₃₀-Alkenylgruppen umfassen z. B. n-Octenyl, n-Nonenyl, n-Decenyl, n-Undecenyl, n-Dodecenyl, n-Tridecenyl, n-Tetradecenyl, n-Pentadecenyl, n-Hexadecenyl, n-Heptadecenyl, n-Octadecenyl, n-Nonadecenyl, n-Eicosenyl, n-Docosenyl, n-Tetracosenyl, Hexacosenyl, Triacontenyl, etc.

Die Herstellung der erfindungsgemäßen Polymerzusammensetzungen erfolgt durch radikalische Polymerisation der Monomerzusammensetzung M) in Gegenwart wenigstens einer Polyetherkomponente PE), die in der Regel keine copolymerisierbare Doppelbindung aufweist. Dabei werden spezielle Polymerzusammensetzungen mit vorteilhaften Eigenschaften erhalten. Ohne an eine Theorie gebunden zu sein, kann dies beispielsweise auf eine Wirkung der Polyetherkomponente PE) als Schutzkolloid oder Emulgator zurückzuführen sein. Dies kann beispielsweise auch aus einer zumindest teilweisen Pfropfung auf die Polyetherkomponente als Pfropfgrundlage resultieren. Es sind jedoch auch andere Mechanismen als eine Pfropfung vorstellbar. Die erfindungsgemäßen Polymerzusammensetzungen umfassen ganz allgemein die Verfahrensprodukte der radikalischen Polymerisation, worunter z. B. Pfropfpolymerisate, Homo- und Copolymere der in der Monomermischung M) enthaltenen Monomere, Mischungen von Pfropfpolymerisaten mit ungepfropften Verbindungen der Polyetherkomponente PE) sowie beliebige Mischungen verstanden werden.

Während und im Anschluss an die Polymerisation in Schritt b) werden das Reaktionsgemisch und die in Schritt b) erhaltene Polymerzusammensetzung insbesondere keiner Kondensation unter Entfernung eines niedermolekularen Reaktionsprodukts und/oder in Gegenwart eines Kondensationskatalysators unterzogen. Darunter wird verstanden, dass speziell keine zusätzlichen Maßnahmen durchgeführt werden, mit dem Ziel, den Estergruppengehalt der nach dem erfindungsgemäßen Verfahren erhaltenen Polymerzusammensetzung zu erhöhen.

In einer bevorzugten Ausführung der ersten Variante liegen die erfindungsgemäßen Polymerzusammensetzungen in Form eines transparenten Gels vor. Die Transparenz eines Materials wird bestimmt durch sein Absorptions- und Streuverhalten, d. h. die durchgelassene Lichtmenge und das Erscheinungsbild in der Durchsicht. Die Gesamttransmission (Lichtdurchlässigkeit) ist das Verhältnis von durchgelassenem Licht zu einfallendem Licht. Als Maß für die Lichtdurchlässigkeit wird der Transmissionsgrad τ verwendet: Er ist der Quotient aus dem Lichtstrom Φₙ hinter und Φᵥ vor dem zu prüfenden Material und wird in Prozent angegeben. Dieser Wert enthält neben der Absorbtion auch die Streu- und Reflexionsverluste. Der Transmissionsgrad wird im Allgemeinen in Luft bestimmt und wird als Funktion der Wellenlänge angegeben.

Im Rahmen der Erfindung wird die Lichtdurchlässigkeit (T_{L}) bei einer Wellenlänge von 500 nm bestimmt. Als Bezugsgröße für die maximale Lichtdurchlässigkeit (T_{L}-Wert von 100 %) dient Wasser. Vorzugsweise weist die erfindungsgemäß eingesetzte Polymerzusammensetzung in Form eines transparenten Gels einen T_{L}-Wert, gemessen bei 500 nm, von mindestens 85 %, besonders bevorzugt von mindestens 90 %, bezogen auf die Lichtdurchlässigkeit von Wasser, auf.

### Monomerzusammensetzung M)

Die in Schritt a) bereitgestellte Monomerzusammensetzung M) enthält als Komponente A) wenigstens eine α,β-ethylenisch ungesättigte Säure.

Die in Schritt a) bereitgestellte Monomerzusammensetzung M) besteht bevorzugt aus
- Acrylsäure als α,β-ethylenisch ungesättigte Carbonsäure A1),
- gegebenenfalls wenigstens einer weiteren α,β-ethylenisch ungesättigten Säure A2), und
- 0 bis 0,1 Gew.-%, bezogen auf das Gesamtgewicht der Monomerzusammensetzung M), vernetzend wirkenden Monomeren, die zwei oder mehr als zwei polymerisierbare α,β-ethylenisch ungesättigte Doppelbindungen pro Molekül aufweisen.

Wenn die in Schritt a) bereitgestellte Monomerzusammensetzung wenigstens eine weitere α,β-ethylenisch ungesättigte Säure enthält, ist diese vorzugsweise ausgewählt unter Carbonsäuren, Sulfonsäuren, Phosphonsäuren und Mischungen davon.

In einer ersten bevorzugten Ausführungsform besteht die Komponente A) nur aus Acrylsäure (= Monomer A1).

In einer zweiten bevorzugten Ausführungsform besteht die Komponente A) aus Acrylsäure (= Monomer A1) und wenigstens einer weiteren, davon verschiedenen α,β-ethylenisch ungesättigten Säure (= Monomer A2).

Die weitere α,β-ethylenisch ungesättigte Säure A2) ist vorzugsweise ausgewählt unter Methacrylsäure, Ethacrylsäure, α-Chloracrylsäure, Crotonsäure, Maleinsäure, Itaconsäure, Citraconsäure, Mesaconsäure, Glutaconsäure, Aconitsäure, Fumarsäure, 2-Acrylamido-2-methylpropansulfonsäure, Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, Vinylphosphonsäure, Allylphosphonsäure und Mischungen davon.

Die Säuremonomere A) werden vorzugsweise in protonierter (d. h. nicht neutralisierter) Form zur Polymerisation eingesetzt.

Besonders bevorzugt besteht die Monomerzusammensetzung M) aus Acrylsäure oder Mischungen der Acrylsäure mit Methacrylsäure.

In einer bevorzugten Ausführungsform besteht die Monomerzusammensetzung M) zu mindestens 80 Gew-%, bevorzugt zu mindestens 90 Gew.-%, insbesondere zu mindestens 95 Gew.-%, bezogen auf das Gesamtgewicht der eingesetzten Monomere, aus Acrylsäure.

Insbesondere wird als Monomerzusammensetzung M) ausschließlich Acrylsäure eingesetzt.

### Vernetzer B)

Die erfindungsgemäße Polymerzusammensetzung besteht aus unvernetzten bzw. gering vernetzten Polymeren. Die zur Herstellung der erfindungsgemäßen Polymerzusammensetzung eingesetzte Monomerzusammensetzung M) enthält somit keine oder nur geringe Mengen an vernetzend wirkenden Monomeren B). Vernetzer im Sinne der Erfindung sind Verbindungen mit zwei oder mehr als zwei polymerisierbaren, ethylenisch ungesättigten Doppelbindungen pro Molekül.

Vorzugsweise werden Vernetzer B) in einer Menge von 0 bis 0,1 Gew.-%, besonders bevorzugt 0 bis 0,05 Gew.-%, bezogen auf das Gesamtgewicht der Monomerzusammensetzung M), verwendet. In einer speziellen Ausführungsform enthält die Monomerzusammensetzung M) keine vernetzend wirkenden Monomere B), die zwei oder mehr als zwei polymerisierbare α,β-ethylenisch ungesättigte Doppelbindungen pro Molekül aufweisen.

Geeignete Vernetzer B) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thiopentan-1,5-diol sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer B) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer B) sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer B) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer B) sind ferner geeignet die Acrylsäureamide, Methacrylsäureamide und N-Allylamine von mindestens zweiwertigen Aminen. Solche Amine sind z. B. 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer B) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer B) sind Divinyldioxan, Tetraallylsilan oder Tetravinylsilan.

### Polyetherkomponente PE)

Geeignet als Polyetherkomponente PE) sind Polyetherole mit einem zahlenmittleren Molekulargewicht von wenigstens 200 g/mol und deren Mono- und Di-(C₁-C₆-alkylether).

Geeignete Polyetherole und deren Mono- und Di-(C₁-C₆-alkylether) können linear oder verzweigt, vorzugsweise linear sein. Geeignete Polyetherole und deren Mono- und Di-(C₁-C₆-alkylether)weisen im Allgemeinen ein zahlenmittleres Molekulargewicht im Bereich von etwa 200 bis 100000, bevorzugt 300 bis 50000, besonders bevorzugt 500 bis 40000, auf. Geeignete Polyetherole sind beispielsweise wasserlösliche oder wasserdispergierbare nichtionische Polymere, die Alkylenoxid-Wiederholungseinheiten aufweisen. Vorzugsweise beträgt der Anteil an Alkylenoxid-Wiederholungseinheiten mindestens 30 Gew.-%, bezogen auf das Gesamtgewicht der Verbindung. Geeignete Polyetherole sind Polyalkylenglycole, wie Polyethylenglycole, Polypropylenglycole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Die Alkylenoxidcopolymere können die Alkylenoxideinheiten statistisch verteilt oder in Form von Blöcken einpolymerisiert enthalten. Vorzugsweise beträgt in den Ethylenoxid/Propylenoxid-Copolymeren der Anteil an von Ethylenoxid abgeleiteten Wiederholungseinheiten 40 bis 99 Gew.-%. Besonders bevorzugt als Polyetherkomponente PE) sind Ethylenoxid-Homopolymere und Ethylenoxid/Propylenoxid-Copolymere.

Geeignet als Polyetherkomponente PE) sind weiterhin die Mono- und Di-(C₁-C₂-alkylether) der zuvor beschriebenen Polyetherole. Bevorzugt sind Polyalkylenglycolmonomethylether und Polyalkylenglycoldimethylether.

Geeignet als Polyetherkomponente PE) sind weiterhin Polyethergruppen-haltige Tenside. Geeignet sind allgemein nichtionische und ionische Tenside, die wenigstens eine unpolare und wenigstens eine polare Gruppe aufweisen und die eine Polyethergruppe umfassen.

Die Polyethergruppen-haltigen Tenside PE) sind vorzugsweise ausgewählt unter Alkylpolyoxyalkylenether, Arylpolyoxyalkylenether, Alkylarylpolyoxyalkylenether, alkoxylierte tierische und/oder pflanzliche Fette und/oder Öle, Fettaminalkoxylate, Fettsäureamidalkoxylate, Fettsäurediethanolamidalkoxylate, Polyoxyethylensorbitanfettsäureester, Alkylpolyethersulfate, Arylpolyethersulfate, Alkylarylpolyethersulfate, Alkylpolyethersulfonate, Arylpolyethersulfonate, Alkylarylpolyethersulfonate, Alkylpolyetherphosphateate, Arylpolyetherphosphate, Alkylarylpolyetherphosphate, Glycerinethersulfonate, Glycerinethersulfate, Monoglycerid(ether)sulfate, Fettsäureamidethersulfate, Polyoxyalkylensorbitanfettsäureestern und Mischungen davon.

Zu den bevorzugten nichtionischen Tensiden Polyethergruppen-haltigen Tenside PE) gehören beispielsweise:
- Alkylpolyoxyalkylenether, die sich von niedermolekularen C₃-C₆-Alkoholen oder von C₇-C₃₀-Fettalkoholen ableiten. Dabei kann die Etherkomponente aus Ethylenoxideinheiten, Propylenoxideinheiten, 1,2-Butylenoxideinheiten, 1,4-Butylenoxideinheiten und statistischen Copolymeren und Blockcopolymeren davon abgeleitet sein. Geeignete nichtionische Tenside umfassen unter anderem Tenside der allgemeinen Formel (VI)

   R¹⁰-O-(CH₂CH₂O)ₓ-(CHR¹¹CH₂O)_{y}-R¹² (VI),

   worin R¹⁰ ein linearer oder verzweigter Alkylrest mit 6 bis 22 C-Atomen ist,
   R¹¹ und R¹² unabhängig voneinander Wasserstoff oder ein linearer oder verzweigter Alkylrest mit 1 bis 10 C-Atomen oder H sind, wobei R¹² bevorzugt Methyl ist, und
   x und y unabhängig voneinander 0 bis 300 sind. Bevorzugt ist x = 1 bis 100 und y = 0 bis 30.

   Dazu zählen speziell auch Fettalkoholalkoxylate und Oxoalkoholalkoxylate, wie iso-Tridecylalkohol- und Oleylalkoholpolyoxyethylenether.
- Hydroxylgruppen-haltige Tenside der allgemeinen Formel (VII)

   R¹³-O-(CH₂CH₂O)ₛ-(CH₂CH₂CH₂O)ₜ-(CH₂CH₂CH₂CH₂O)ᵤ-(CH₂CHR¹⁴O)ᵥ-CH₂CH(OH)R¹⁵ (VII)

   wobei
   in den Verbindungen der Formel (VII) die Reihenfolge der Alkylenoxideinheiten beliebig ist,
   s, t, u und v unabhängig voneinander für eine ganze Zahl von 0 bis 500 stehen, wobei die Summe aus s, t, u und v > 0 ist,
   R¹³ und R¹⁵ unabhängig voneinander für einen geradkettigen oder verzweigten, gesättigten C₁-C₄₀-Alkylrest oder einen ein- oder mehrfach ungesättigten C₂-C₄₀-Alkenylrest stehen, und
   R¹⁴ ausgewählt ist unter Methyl, Ethyl, n-Propyl, Isopropyl oder n-Butyl.

   In den Verbindungen der allgemeinen Formel (VII) steht die Summe aus s, t, u und v vorzugsweise für einen Wert von 10 bis 300, besonders bevorzugt von 15 bis 200 und insbesondere von 20 bis 150.
   Bevorzugt stehen t und u für 0. Dann steht die Summe aus s und v vorzugsweise für einen Wert von 10 bis 300, besonders bevorzugt von 15 bis 200 und insbesondere von 20 bis 150.
   In den Verbindungen der allgemeinen Formel (VII) stehen vorzugsweise R¹³ und R¹⁵ unabhängig voneinander für einen geradkettigen oder verzweigten, gesättigten C₂-C₃₀-Alkylrest. Dabei können R¹³ und R¹⁵ auch für Mischungen verschiedener Alkylreste stehen.
   In den Verbindungen der allgemeinen Formel (VII) steht R¹⁴ vorzugsweise für Methyl oder Ethyl, insbesondere für Methyl.
   Eine bevorzugte Ausführung sind Hydroxylgruppen-haltige Tenside der allgemeinen Formel (VII.1)

   R¹³-O-(CH₂CH₂O)ₛ-(CH₂CH(CH₃)O)ᵥ-CH₂CH(OH)R¹⁵ (VII.1)

   wobei
   die Reihenfolge der -(CH₂CH₂O)- und der (CH₂CH(CH₃)O)-Einheiten beliebig ist,
   s und v unabhängig voneinander für eine ganze Zahl von 0 bis 500 stehen, wobei die Summe aus s und v > 0 ist, und
   R¹³ und R¹⁵ unabhängig voneinander für einen geradkettigen, gesättigten C₁-C₃₀-Alkylrest oder einen verzweigten, gesättigten C₃-C₃₀-Alkylrest oder einen ein- oder mehrfach ungesättigten C₂-C₃₀-Alkenylrest stehen.

   In den Verbindungen der allgemeinen Formel (VII.1) steht die Summe aus s und v vorzugsweise für einen Wert von 10 bis 300, besonders bevorzugt von 15 bis 200 und insbesondere von 20 bis 150.
   Zur Gruppe dieser nichtionischen Tenside zählen beispielsweise Hydroxymischether der allgemeinen Formel (C₆₋₂₂-Alkyl)-CH(OH)CH₂O-(EO)₂₀₋₁₂₀-(C₂₋₂₆-Alkyl).
- Alkoholpolyoxyalkylenester der allgemeinen Formel (VIII)

   R¹⁶-O-(CH₂CH₂O)ₚ-(CH₂CHR¹⁷O)_{q}-C(=O)R¹⁸ (VIII)

   wobei
   in den Verbindungen der Formel (VIII) die Reihenfolge der Alkylenoxideinheiten beliebig ist,
   p und q unabhängig voneinander für eine ganze Zahl von 0 bis 500 stehen, wobei die Summe aus p und q > 0 ist,
   R¹⁶ und R¹⁸ unabhängig voneinander für einen geradkettigen oder verzweigten, gesättigten C₁-C₄₀-Alkylrest oder einen ein- oder mehrfach ungesättigten C₂-C₄₀-Alkenylrest stehen, und
   R¹⁷ ausgewählt ist unter Methyl, Ethyl, n-Propyl, Isopropyl oder n-Butyl.

   In den Verbindungen der allgemeinen Formel (VIII) steht die Summe aus p und q vorzugsweise für einen Wert von 10 bis 300, besonders bevorzugt von 15 bis 200 und insbesondere von 20 bis 150.
   In den Verbindungen der allgemeinen Formel (VIII) stehen vorzugsweise R¹⁶ und R¹⁸ unabhängig voneinander für einen geradkettigen oder verzweigten, gesättigten C₄-C₃₀-Alkylrest. Dabei können R¹⁶ und R¹⁸ auch für Mischungen verschiedener Alkylreste stehen.
   In den Verbindungen der allgemeinen Formel (VIII) steht R¹⁷ vorzugsweise für Methyl oder Ethyl, insbesondere für Methyl.
   Dazu zählt beispielsweise Laurylalkoholpolyoxyethylenacetat.
- Alkylarylalkoholpolyoxyethylenether, z. B. Octylphenol-polyoxyethylenether,
- alkoxylierte tierische und/oder pflanzliche Fette und/oder Öle, beispielsweise Maisölethoxylate, Rizinusölethoxylate, Talgfettethoxylate,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Polyoxyalkylensorbitanfettsäureester.

Ein Beispiel für ein Alkylpolyethersulfat ist Natriumdodecylpoly(oxyethylen)sulfat (Natriumlaurylethersulfat, SLES).

### Lösungsmittel LM)

Die radikalische Polymerisation in Schritt b) kann in Gegenwart eines Lösungsmittels LM) erfolgen, das ausgewählt ist unter Wasser, C₁-C₆-Alkanolen, von PE) verschiedenen Polyolen, deren Mono- und Dialkylethern und Mischungen davon. Geeignete Polyole und deren Mono- und Dialkylether umfassen auch Alkylenglycolmono(C₁-C₄-alkyl)ether, Alkylenglycoldi(C₁-C₄-alkyl)ether, Oligoalkylenglycole mit einem zahlenmittleren Molekulargewicht von weniger als 200 g/mol und deren Mono(C₁-C₄-alkyl)ether und Di(C₁-C₄-alkyl)ether.

Das Lösungsmittel LM) ist vorzugsweise ausgewählt unter Wasser, Methanol, Ethanol, n-Propanol, Isopropanol, n-Butanol, Ethylenglycol, Ethylenglycolmono(C₁-C₄-alkyl)-ethern, Ethylenglycoldi(C₁-C₄-alkyl)ethern, 1,2-Propylenglycol, 1,2-Propylenglycolmono(C₁-C₄-alkyl)ethern, 1,2-Propylenglycoldi(C₁-C₄-alkyl)ethern Glycerin, Polyglycerinen, Oligoalkylenglycolen, Oligoalkylenglycolmono(C₁-C₄-alkyl)ethern, Oligoalkylenglycoldi(C₁-C₄-alkyl)ethern, wobei die vier letztgenannten ein zahlenmittleres Molekulargewicht von weniger als 200 g/mol aufweisen, und Mischungen davon.

Geeignete Oligoethylenglycole sind unter den CTFA-Bezeichnungen PEG-6, PEG-8, PEG-12, PEG-6-32, PEG-20, PEG-150, PEG-7M, PEG-12M and PEG-115M kommerziell erhältlich. Dazu zählen speziell die Pluriol E ® Marken der BASF SE. Geeignete Alkylpolyalkylenglycole sind die entsprechenden Pluriol A ...E ® Marken der BASF SE. Bevorzugt sind die isomeren Dipropylenglycole, wie 1,1'-Oxydi-2-propanol, 2,2'-Oxydi-1-propanol, 2(2-Hydroxypropoxy)-1-propanol und Mischungen davon.

Das Lösungsmittel LM) ist besonders bevorzugt ausgewählt unter Wasser, Ethanol, n-Propanol, Isopropanol, Ethylenglycol, Diethylenglycol, 1,2-Propylenglycol, Dipropylenglycolen und Mischungen davon.

In einer speziellen Ausführungsform erfolgt die radikalische Polymerisation in Schritt b) in Gegenwart eines Lösungsmittels LM), das Propylenglycol und/oder Dipropylenglycol enthält.

In einer weiteren speziellen Ausführungsform erfolgt die radikalische Polymerisation in Schritt b) in Gegenwart eines Lösungsmittels LM), das aus Propylenglycol und/oder Dipropylenglycol und gegebenenfalls Wasser besteht. Ganz speziell erfolgt die radikalische Polymerisation in Schritt b) in Gegenwart eines Lösungsmittels LM), das aus Propylenglycol und/oder Dipropylenglycol besteht.

Bevorzugt enthält die Polymerzusammensetzung das Lösungsmittel LM) in einer Menge von 0 bis 50 Gew.-%, bevorzugt 0,1 bis 40 Gew.-%, bezogen auf das Gesamtgewicht der Polymerzusammensetzung.

### Herstellung der erfindungsgemäß verwendeten Polymerzusammensetzungen

Die Herstellung der erfindungsgemäß verwendeten Polymerzusammensetzungen umfasst eine radikalische Polymerisation der Monomerzusammensetzung M) in Gegenwart wenigstens einer Polyetherkomponente PE). Sie wird vorzugsweise im Zulaufverfahren durchgeführt. Dabei werden im Allgemeinen zumindest die Monomere in flüssiger Form dem Reaktionsansatz zudosiert. Unter den Dosierbedingungen flüssige Monomere können dem Reaktionsansatz ohne Zugabe eines Lösungsmittels LM) zugeführt werden, ansonsten werden die Monomere als Lösung in einem geeigneten Lösungsmittel LM) eingesetzt.

Die Dosierraten des Monomerzulaufs/der Monomerzuläufe sowie etwaiger weiterer Zuläufe (Initiator, Regler, etc.) werden vorzugsweise so gewählt, dass die Polymerisation mit der gewünschten Umsatzrate aufrechterhalten wird. Die Zugabe der einzelnen Zuläufe kann dabei kontinuierlich, periodisch, mit konstanter oder wechselnder Dosierrate, im Wesentlichen zeitgleich oder zeitversetzt erfolgen. Vorzugsweise erfolgt die Zugabe aller Zuläufe zum Reaktionsansatz kontinuierlich.

Bevorzugt umfasst die radikalische Polymerisation in Schritt b) des erfindungsgemäßen Verfahrens
b1) Bereitstellung einer Vorlage, die wenigstens einen Teil der Polyetherkomponente PE), gegebenenfalls wenigstens einen Teil des Reglers R) und, falls die Polymerisation in Gegenwart eines Lösungsmittels LM) erfolgt, gegebenenfalls wenigstens einen Teil von LM) enthält;
b2) Zugabe der Monomerzusammensetzung M) in einem Zulauf oder in mehreren Zuläufen sowie Zugabe eines Zulaufs, der den Radikalstarter S), gelöst in einem Teil der wenigstens einen Polyetherkomponente PE) und/oder des Lösungsmittels LM) enthält und gegebenenfalls Zugabe eines Zulaufs, der die Menge des Reglers R) enthält, die nicht in der Vorlage eingesetzt wird;
b3) gegebenenfalls Nachpolymerisierung der in Schritt b2) erhaltenen Reaktionsmischung.

Üblicherweise wird die Vorlage vor der Zugabe der Zuläufe unter Rühren auf die Polymerisationstemperatur erwärmt.

Bevorzugt werden die einzelnen Reaktanten simultan in getrennten Zuläufen zugegeben, wobei die Flussraten der Zuläufe in der Regel über den Zeitraum der Zugabe möglichst konstant gehalten werden.

Die Zugabe der Zuläufe in Schritt b2) erfolgt über einen Zeitraum der in vorteilhafter Weise so gewählt wird, dass die bei der exothermen Polymerisationsreaktion entstehende Reaktionswärme ohne größeren technischen Aufwand, z. B. ohne die Verwendung eines Rückflusskühlers, abgeführt werden kann. Üblicherweise erfolgt die Zugabe der Zuläufe über einen Zeitraum von 1 bis 10 Stunden. Bevorzugt erfolgt die Zugabe der Zuläufe über einen Zeitraum von 2 bis 8 Stunden, besonders bevorzugt über 3 bis 4 Stunden.

Während der radikalischen Polymerisation werden in der Regel das optional eingesetzte Lösungsmittel und/oder etwaig entstehende Kondensationsprodukte nicht abgeführt. D. h. während der Polymerisation findet üblicherweise kein oder ein im Rahmen der technischen Möglichkeiten nur sehr geringer Stoffaustausch mit der Umgebung statt.

Die Polymerisation kann in der Regel bei Umgebungsdruck oder vermindertem oder erhöhtem Druck erfolgen. Bevorzugt wird die Polymerisation bei Umgebungsdruck durchgeführt.

Die Polymerisation erfolgt in der Regel bei konstanter Temperatur, kann aber auch bei Bedarf während der Polymerisation variiert werden. Bevorzugt wird die Polymerisationstemperatur über den gesamten Reaktionszeitraum, d. h. der Schritte b2) und b3), möglichst konstant gehalten. Je nachdem welche Einsatzstoffe im erfindungsgemäßen Verfahren eingesetzt werden, bewegt sich die Polymerisationstemperatur üblicherweise im Bereich von 10 bis 150 °C. Bevorzugt erfolgt die Polymerisation in Schritt b) bei einer Temperatur im Bereich von 20 bis 150 °C, bevorzugt von 30 bis 120 °C, insbesondere von 40 bis 90 °C. Sofern die Polymerisation nicht unter erhöhtem Druck durchgeführt wird und der Reaktionsmischung wenigstens ein optionales Lösungsmittel LM) zugesetzt wurde, bestimmt das Lösungsmittel bzw. Lösungsmittelgemisch durch die entsprechenden Siedetemperaturen die maximale Reaktionstemperatur.

Die Polymerisation kann in Abwesenheit oder in Gegenwart eines Inertgases erfolgen. Unter Inertgas wird in der Regel ein Gas verstanden, welches unter den gegebenen Reaktionsbedingungen keine Reaktion mit den an der Reaktion beteiligten Edukten, Reagenzien, Lösungsmitteln oder den entstehenden Produkten eingeht. Dazu zählt z. B. Stickstoff, Argon, etc.

Falls die Polymerisation in Gegenwart eines Lösungsmittels durchgeführt wird, so ist dieses ausgewählt unter den zuvor beschriebenen Lösungsmitteln LM).

Zur Herstellung der Polymerisate können die Monomeren mit Hilfe von Radikale bildenden Initiatoren, im Nachfolgenden auch als Radikalstarter oder Starter bezeichnet, polymerisiert werden. Als Radikalstarter (Initiatoren) für die radikalische Polymerisation kommen grundsätzlich alle Radikalstarter in Frage, die im Reaktionsmedium, wie es zum Zeitpunkt ihrer Zugabe vorherrscht, im Wesentlichen löslich sind und bei den gegebenen Reaktionstemperaturen eine ausreichende Aktivität besitzen, um die Polymerisation zu starten. In das erfindungsgemäße Verfahren kann ein einzelner Radikalstarter oder eine Kombination wenigstens zweier Radikalstarter eingesetzt werden. Im letzteren Fall können die wenigstens zwei Radikalstarter im Gemisch oder vorzugsweise getrennt, gleichzeitig oder nacheinander, z. B. zu unterschiedlichen Zeitpunkten im Reaktionsverlauf, eingesetzt werden.

Als Radikalstarter für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, tert.-Butylperoctoat, Dilauroylperoxid, tert.-Butylperisobutyrat, tert.-Butylperacetat, Di-tert.-amylperoxid, tert.-Butylhydroperoxid, 2,2'-Azo-bis-isobutyronitril, Azo-bis-(2-amidinopropan)dihydrochlorid, Azobis(2,4-dimethylvaleronitril) oder 2,2'-Azo-bis-(2-methyl-butyronitril).

Geeignet sind auch Initiatormischungen oder Redox-Initiator-Systeme, wie z. B. Ascorbinsäure/Eisen(II)sulfat/Natriumperoxodisulfat,
tert.-Butylhydroperoxid/Natriumdisulfit,
tert.-Butylhydroperoxid/Natriumhydroxymethansulfinat,
H₂O₂/Cu^{l}.

In dem erfindungsgemäßen Verfahren bewegt sich die Menge an eingesetztem Initiatorsystem (Starter) im Bereich von 0,01 bis 10 Gew.-%, bevorzugt im Bereich von 0,05 bis 5 Gew.-%, besonders bevorzugt im Bereich von 0,1 bis 2 Gew.-%.

Im erfindungsgemäßen Verfahren wird der Radikalstarter in der Regel als Lösung in einem Lösungsmittel bereitgestellt, das wenigstens eines der zuvor genannten Lösungsmittel LM) umfasst.

Die Menge an Regler, die üblicherweise in dem erfindungsgemäßen Verfahren eingesetzt wird, beträgt 1 bis 40 pphm ("parts per hundred monomer", d. h. Gewichtsteile, bezogen auf hundert Gewichtsteile Monomerzusammensetzung). Bevorzugt liegt die in dem erfindungsgemäßen Verfahren eingesetzte Menge an Regler im Bereich von 1 bis 30 pphm, besonders bevorzugt im Bereich von 2 bis 20 pphm.

Als Regler (Polymerisationsregler) werden allgemein Verbindungen mit hohen Übertragungskonstanten bezeichnet. Regler beschleunigen Kettenübertragungsreaktionen und bewirken damit eine Herabsetzung des Polymerisationsgrades der resultierenden Polymeren, ohne die Bruttoreaktions-Geschwindigkeit zu beeinflussen. Bei den Reglern kann man zwischen mono-, bi- oder polyfunktionalen Reglern unterscheiden, je nach Anzahl der funktionellen Gruppen im Molekül, die zu einer oder mehreren Kettenübertragungsreaktionen führen können. Geeignete Regler werden beispielsweise ausführlich beschrieben von K. C. Berger und G. Brandrup in J. Brandrup, E. H. Immergut, Polymer Handbook, 3. Aufl., John Wiley & Sons, New York, 1989, S. II/81-II/141.

Als Regler eignen sich beispielsweise Aldehyde wie Formaldehyd, Acetaldehyd, Propionaldehyd, n-Butyraldehyd, Isobutyraldehyd.

Ferner können auch als Regler eingesetzt werden: Ameisensäure, ihre Salze oder Ester, wie Ammoniumformiat, 2,5-Diphenyl-1-hexen, Hydroxylammoniumsulfat, und Hydroxylammoniumphosphat.

Weitere geeignete Regler sind Halogenverbindungen, z. B. Alkylhalogenide, wie Tetrachlormethan, Chloroform, Bromtrichlormethan, Bromoform, Allylbromid und Benzylverbindungen wie Benzylchlorid oder Benzylbromid.

Weitere geeignete Regler sind Allylverbindungen, wie z. B. Allylalkohol, funktionalisierte Allylether, wie Allylethoxylate, Alkylallylether oder Glycerinmonoallylether.

Bevorzugt werden als Regler Verbindungen eingesetzt, die Schwefel in gebundener Form enthalten.

Verbindungen dieser Art sind beispielsweise anorganische Hydrogensulfite, Disulfite und Dithionite oder organische Sulfide, Disulfide, Polysulfide, Sulfoxide und Sulfone. Dazu zählen Di-n-butylsulfid, Di-n-octylsulfid, Diphenylsulfid, Thiodiglykol, Ethylthioethanol, Diisopropyldisulfid, Di-n-butyldisulfid, Di-n-hexyldisulfid, Diacetyldisulfid, Diethanolsulfid, Di-t-butyltrisulfid, Dimethylsulfoxid, Dialkylsulfid, Dialkyldisulfid und/oder Diarylsulfid.

Geeignet als Polymerisationsregler sind weiterhin Thiole (Verbindungen, die Schwefel in Form von SH-Gruppen erhalten, auch als Mercaptane bezeichnet). Bevorzugt sind als Regler mono-, bi- und polyfunktionale Mercaptane, Mercaptoalkohole und/oder Mercaptocarbonsäuren. Beispiele für diese Verbindungen sind Allylthioglykolate, Ethylthioglykolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Thioessigsäure, Thioharnstoff und Alkylmercaptane wie n-Butylmercaptan, n-Hexylmercaptan oder n-Dodecylmercaptan.

Beispiele für bifunktionale Regler, die zwei Schwefelatome in gebundener Form enthalten, sind bifunktionale Thiole, wie z. B. Dimercaptopropansulfonsäure (Natriumsalz), Dimercaptobernsteinsäure, Dimercapto-1-propanol, Dimercaptoethan, Dimercaptopropan, Dimercaptobutan, Dimercaptopentan, Dimercaptohexan, Ethylenglykol-bis-thioglykolate und Butandiol-bis-thioglykolat. Beispiele für polyfunktionale Regler sind Verbindungen, die mehr als zwei Schwefelatome in gebundener Form enthalten. Beispiele hierfür sind trifunktionale und/oder tetrafunktionale Mercaptane.

Alle genannten Regler können einzeln oder in Kombination miteinander eingesetzt werden.

Üblicherweise wird der Regler entweder teilweise zur Vorlage, d. h. vor der eigentlichen Polymerisation, zugegeben oder er wird vollständig in Schritt b2) über einen der Zuläufe kontinuierlich zur Polymerisationsmischung zugegeben.

Die Menge des Reglers und die Art wie dieser zur Reaktionsmischung zugegeben wird, haben einen starken Einfluss auf das mittlere Molekulargewicht der Polymerzusammensetzung. Wenn weniger Regler eingesetzt wird und/oder wenn die Zugabe größtenteils oder vollständig während der Polymerisation stattfindet (Schritt b2), führt dies in der Regel zu einem größeren mittleren Molekulargewicht als wenn größere Mengen Regler verwendet werden und/oder der Regler teilweise in der Vorlage eingesetzt wird.

Das Mengenverhältnis der zur Herstellung der Polymerzusammensetzung verwendeten Polyetherkomponente PE) zur eingesetzten Monomerzusammensetzung M) liegt vorzugsweise in einem Bereich von 1,0 : 0,8 bis 1,0 : 5.

Vorzugsweise beträgt die Menge an Polyetherkomponente PE) in der Vorlage in Schritt b1) 10 bis 100 Gew.-%, besonders bevorzugt 25 bis 100 Gew.-% und insbesondere 30 bis 100 Gew.-%, bezogen auf das Gesamtgewicht der zur Polymerisation eingesetzten Polyetherkomponente PE).

Bevorzugt beträgt der Gehalt an Lösungsmittel in der Vorlage in Schritt b1) maximal 90 Gew.-%, bezogen auf das Gesamtgewicht der im Vorlauf befindlichen Einsatzstoffe. Besonders bevorzugt beträgt der Gehalt an Lösungsmittel in der Vorlage in Schritt b1) maximal 75 Gew.-%, insbesondere maximal 70 Gew.-%, bezogen auf das Gesamtgewicht der in der Vorlage befindlichen Einsatzstoffe. Die Lösungsmittelmenge ändert sich über den gesamten Verlauf des Verfahrens in der Regel nur um wenige Gewichtsprozente.

Üblicherweise werden Lösungsmittel LM) verwendet, die einen Siedepunkt bei Normaldruck (1 bar) von unter 240 °C aufweisen. Bevorzugt ist das Lösungsmittel LM) ausgewählt unter Wasser, Ethylenglycol, Ethylenglycolmono(C₁-C₄-alkyl)ethern, Ethylenglycoldi(C₁-C₄-alkyl)ethern, 1,2-Propylenglycol, 1,2-Propylenglycolmono(C₁-C₄-alkyl)ethern, 1,2-Propylenglycoldi(C₁-C₄-alkyl)ethern, Dipropylenglycol und Mischungen davon. Besonders bevorzugt ist das Lösungsmittel LM) ausgewählt unter Ethylenglycol, 1,2-Propylenglycol, Dipropylenglycol oder Mischungen davon. In einer speziellen Ausführung ist das Lösungsmittel LM) ausgewählt unter Mischungen von Wasser mit wenigstens einem wassermischbaren Lösungsmittel, vorzugsweise ausgewählt unter Ethylenglycol, 1,2-Propylenglycol und Dipropylenglycol, mit der Maßgabe, dass die Mischung maximal 10 Gew.-% Wasser enthält. In einer weiteren speziellen Ausführung ist das Lösungsmittel LM) ausgewählt unter 1,2-Propylenglycol, Dipropylenglycol sowie Mischungen davon.

In einer speziellen Variante enthält die in Schritt b1) bereitgestellte Vorlage kein Lösungsmittel. Dieses wird erst in Schritt b2) über wenigstens einen der Zuläufe zugegeben. In einer ganz speziellen Variante wird kein Lösungsmittel vorgelegt sowie über den gesamten Verlauf des Verfahrens kein Lösungsmittel zugegeben.

Bevorzugt werden die nach Beendigung der Polymerisation (Schritt b3)) erhaltenen Polymerzusammensetzungen in ein geeignetes Gefäß transferiert und gegebenenfalls direkt auf Umgebungstemperatur (20 °C) abgekühlt.

In der Regel besitzt die nach dem zuvor beschriebenen Verfahren erhaltene Polymerzusammensetzung bereits eine gelartige Konsistenz. Gewünschtenfalls können die rheologischen Eigenschaften der Polymerzusammensetzung beispielsweise durch die Zugabe von Lösungsmitteln und/oder Verdickern eingestellt werden. Geeignete Verdicker sind die im Folgenden beschriebenen Verbindungen f).

Die erfindungsgemäß verwendete Polymerzusammensetzung ist vorzugsweise transparent, d. h. sie weist eine Lichtdurchlässigkeit (T_{L}-Wert) von mindestens 85 %, insbesondere von mindestens 90 %, bezogen auf die Lichtdurchlässigkeit von Wasser, auf.

Die erfindungsgemäß verwendete Polymerzusammensetzung weist vorzugsweise eine Viskosität in einem Bereich von etwa 600 bis 10 000 000 mPas mPas, besonders bevorzugt von 1000 bis 1 000 000 mPas, insbesondere von 2 000 bis 500 000 mPas, bei 20°C auf. Die Viskosität bei 20°C sowie der Viskositätsverlauf in Abhängigkeit der Temperatur der zu untersuchenden Proben wurde mittels eines Rotationsrheometers (DHR-1 der Firma TA-Instruments mit Peltiersystem, Platte/Platte-Geometrie, Ø 40 mm, h = 1 mm) bei Temperaturen von 20 °C bis 80 °C untersucht. Temperatur-Rampe (γ = 1 % mit *M*ₘᵢₙ = 100 µNm). Messtemperatur(en) von 80 °C zu 20 °C und zurück je zwei Läufe (Kühl-/Heizrate 2 K/min). Messzeit je 30 min.

Die erfindungsgemäß verwendete Polymerzusammensetzung weist vorzugsweise einen Gehalt an Säuregruppen von mehr als 1 mmol/g, besonders bevorzugt von mehr als 1.3 mmol/g auf. Die erfindungsgemäße Polymerzusammensetzung weist vorzugsweise einen Gehalt an Säuregruppen von höchstens 15 mmol/g auf. Die erfindungsgemäße Polymerzusammensetzung weist insbesondere einen Gehalt an Säuregruppen von 1.5 mmol/g bis 15 mmol/g auf.

Die erfindungsgemäß verwendete Polymerzusammensetzung weist vorzugsweise einen Gehalt an Säuregruppen von 3 mmol/g bis 8 mmol/g auf.

Die Säuregruppen der erfindungsgemäß verwendeten Polymerzusammensetzung können nicht, teilweise oder vollständig neutralisiert sein. Bevorzugt sind die Säuregruppen der erfindungsgemäßen Polymerzusammensetzung nicht oder nur teilweise neutralisiert.

Die Säuregruppen der erfindungsgemäßen Polymerzusammensetzung liegen vorzugsweise in nicht neutralisierter Form vor.

Vorzugsweise weist die Polymerzusammensetzung eine Löslichkeit in Wasser bei 40 °C und einen pH-Wert von 8 von mindesten 5 g/L auf.

Das gewichtsmittlere Molekulargewicht M_{w} der erfindungsgemäßen Polymerzusammensetzung, bestimmt mittels Gelpermeationschromatographie (GPC) unter Verwendung von neutralisierter Polyacrylsäure als Polymerstandard, beträgt vorzugsweise 1000 bis 70000 Dalton.

### Formulierungen für die maschinelle Geschirrreinigung

Die erfindungsgemäß verwendete Polymerzusammensetzung hat sowohl eine Wirkung als Tensid als auch als Cobuilder. Beispiele für erfindungsgemäße Formulierungen, die die Polymerzusammensetzung enthalten, für die maschinelle Geschirrreinigung, umfassen somit Maschinengeschirrspülmittel, Klarspülmittel und Maschinengeschirrspülmittel mit Klarspülfunktion. Die Formulierungen liegen insbesondere bei Raumtemperatur (20 °C) gelförmig, fest oder in Anteilen gelförmig und fest vor.

Die erfindungsgemäßen Formulierungen für die maschinelle Geschirrreinigung zeichnen sich insbesondere durch eine hervorragende belagsinhibierende Wirkung beim Einsatz im Klarspülgang des maschinellen Geschirrspülers aus (d. h. sie wirkt als Inkrustierungsinhibitor). Sie wirken dabei sowohl gegenüber anorganischen als auch gegenüber organischen Belägen inhibierend. Bei den anorganischen Belägen handelt es sich insbesondere um Calcium- und Magnesiumphosphat, -carbonat, -silikat und/oder -phosphonat, die aus den im Wasser enthaltenen Calcium- und Magnesiumsalzen und den in üblichen Geschirrspülmitteln enthaltenen Buildern entstehen. Bei den organischen Belägen handelt es sich insbesondere um Schmutzbestandteile aus der Spülflotte, wie z. B. Eiweiß-, Stärke- und Fettbeläge. Die erfindungsgemäßen Formulierungen für die maschinelle Geschirrreinigung sind auch gegenüber sogenannten Carry-Over-Belägen wirksam, die aus der Restwassermenge im Sumpf der Spülmaschine stammen und u. a. Geschirrspülmittelreste und eventuell auch noch Schmutzreste aus dem vorhergehenden Waschgang der Geschirrspülmaschine enthalten.

Die Bestandteile der Polymerzusammensetzung unterstützen weiterhin die Reinigungsleistung der Gesamtformulierung, indem sie den Schutz ablösen und dispergieren.

Die erfindungsgemäße Formulierung für die maschinelle Geschirrreinigung umfasst vorzugsweise folgende Bestandteile:
a) wenigstens eine erfindungsgemäße gelförmige Polymerzusammensetzung,
b) wenigstens einen Builder (auch als Sequestrierungsmittel, Gerüststoff, Komplexbildner, Chelator, Chelatisierungsmittel oder Enthärter bezeichnet),
c) gegebenenfalls wenigstens ein Enzym,
d) gegebenenfalls wenigstens ein Bleichmittel,
e) Wasser,
f) gegebenenfalls wenigstens einen Verdicker, und
g) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Löslichkeitsvermittlern und organischen Lösungsmitteln.

Die erfindungsgemäße gelförmige Formulierung für die maschinelle Geschirrreinigung enthält, bezogen auf das Gesamtgewicht der Formulierung, vorzugsweise:
a) 0,1 bis 50 Gew.-% wenigstens einer erfindungsgemäßen gelförmigen Polymerzusammensetzung,
b) 5 bis 90 Gew.-% wenigstens eines Builders und/oder Cobuilders,
c) 0 bis 8 Gew.-% wenigstens eines Enzyms,
d) 0 bis 30 Gew.-% wenigstens eines Bleichmittels,
e) 0,1 bis 90 Gew.-% Wasser,
f) 0 bis 8 Gew.-% wenigstens eines Verdickers
g) 0 bis 25 Gew.-% wenigstens eines weiteren Zusatzstoffs,
mit der Maßgabe, dass sich die Gewichtsmengen der Komponenten a) bis g) zu 100 Gew.-% ergänzen.

In einer bevorzugten Ausführungsform enthält die erfindungsgemäße gelförmige Formulierung für die maschinelle Geschirrreinigung wenigstens ein Enzym.

Die erfindungsgemäße Formulierung für die maschinelle Geschirrreinigung enthält, bezogen auf das Gesamtgewicht der Formulierung, vorzugsweise:
a) 2 bis 40 Gew.-% wenigstens einer erfindungsgemäßen gelförmigen Polymerzusammensetzung,
b) 5 bis 80 Gew.-% wenigstens eines Builders und/oder Cobuilders,
c) 0,1 bis 6 Gew.-% wenigstens eines Enzyms,
d) 0 bis 30 Gew.-% wenigstens eines Bleichmittels,
e) 0,1 bis 80 Gew.-% Wasser,
f) 0 bis 6 Gew.-% wenigstens eines Verdickers
g) 0 bis 25 Gew.-% wenigstens eines weiteren Zusatzstoffs,
mit der Maßgabe, dass sich die Gewichtsmengen der Komponenten a) bis g) zu 100 Gew.-% ergänzen.

Besonders bevorzugt enthält die erfindungsgemäße Formulierung für die maschinelle Geschirrreinigung, bezogen auf das Gesamtgewicht der Formulierung:
a) 0,12 bis 30 Gew.-% wenigstens einer erfindungsgemäßen gelförmigen Polymerzusammensetzung,
b) 5 bis 75 Gew.-% wenigstens eines Builders und/oder Cobuilders,
c) 0,1 bis 6 Gew.-% wenigstens eines Enzyms,
d) 0 bis 25 Gew.-% wenigstens eines Bleichmittels,
e) 0,1 bis 80 Gew.-% Wasser,
f) 0,1 bis 5 Gew.-% wenigstens eines Verdickers
g) 0 bis 25 Gew.-% wenigstens eines weiteren Zusatzstoffs,
mit der Maßgabe, dass sich die Gewichtsmengen der Komponenten a) bis g) zu 100 Gew.-% ergänzen.

Die Polymerzusammensetzung a) kann in die Formulierung eingearbeitet werden oder in der Gesamtformulierung in einem separaten Bereich als klare Gelkomponente von den übrigen Komponenten separat vorliegen (z. B. abgetrennt durch eine Folie, beispielsweise aus Polyvinylalkohol).

Die Reinigerformulierungen können als Tabletten oder vollständig flüssige Formulierungen vorliegen. Es können aber auch mit Folie oder Formkörper hergestellte Behälter mit 1 bis 6 gleich großen oder unterschiedlich großen Einzelabschnitten vorliegen. Diese können unabhängig voneinander mit Pulver, Granulat, Feststoff oder Flüssigkeit gefüllt sein. Die Polymerzusammensetzung a) wird bevorzugt in einem separaten Kompartiment abgefüllt und liegt dort als klares Gel vor. Die Polymerzusammensetzung a) kann zusätzlich verdickt oder eingefärbt werden.

### Komponente a)

Bezüglich als Komponente a) geeignete und bevorzugte erfindungsgemäße Polymerzusammensetzungen wird auf die vorstehenden Ausführungen verwiesen.

### Komponente b)

Builder und Cobuilder, die teilweise auch als Sequestrierungsmittel, Gerüststoff, Komplexbildner, Chelator, Chelatisierungsmittel oder Enthärter bezeichnet werden, binden Erdalkalimetalle und andere wasserlösliche Metallsalze. Sie helfen Schmutz aufzubrechen, dispergieren Schmutzteile, helfen Schmutz abzulösen und haben teilweise eine eigene Waschwirkung. Außerdem halten sie, wenn sie fest sind und in pulverförmigen Formulierungen eingesetzt werden, das Pulver rieselfähig.

Geeignete Builder können sowohl organischer als auch anorganischer Natur sein. Beispiele sind Alumosilikate, Carbonate, Phosphate und Polyphosphate, Polycarbonsäuren, Polycarboxylate, Hydroxycarbonsäuren, Phosphonsäuren, z. B. Hydroxyalkylphosphonsäuren, Phosphonate, Aminopolycarbonsäuren und deren Salze und polymere Carbonsäuregruppen-haltige Verbindungen und deren Salze.

Als Builder geeignete kristalline Silikate sind beispielsweise Disilikate oder Schichtsilikate, z. B. 5-Na₂Si₂O₅ oder B-Na₂Si₂O₅ (SKS 6 bzw. SKS 7). Die Silikate können in Form ihrer Alkalimetall-, Erdalkalimetall- oder Ammoniumsalze eingesetzt werden, vorzugsweise als Na-, Li- und Mg-Silikate. Amorphe Silikate, wie beispielsweise Natriummetasilikat, welches eine polymere Struktur aufweist, oder amorphes Disilikat (Britesil® H 20 Hersteller: Akzo) sind ebenfalls verwendbar. Bevorzugt ist hierunter Natriumdisilikat.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt.

Übliche, als anorganische Builder eingesetzte Phosphate sind Alkalimetallorthophosphate und/oder -polyphosphate wie z. B. Pentanatriumtriphosphat.

Geeignete organische Builder sind beispielsweise C₄-C₃₀-Di-, -Tri- und -Tetracarbonsäuren, wie z. B. Bernsteinsäure, Propantricarbonsäure, Butantetracarbonsäure, Cyclopentantetracarbonsäure und Alkyl- und Alkenylbernsteinsäuren mit C₂-C₂₀-Alkyl- bzw. -Alkenyl-Resten.

Geeignete organische Builder sind weiterhin Hydroxycarbonsäuren und Polyhydroxycarbonsäuren (Zuckersäuren). Dazu zählen C₄-C₂₀-Hydroxycarbonsäuren wie z. B. Äpfelsäure, Weinsäure, Gluconsäure, Schleimsäure, Milchsäure, Glutarsäure, Citronensäure, Tartronsäure, Glucoheptonsäure, Lactobionsäure und Saccharosemono-, -di- und -tricarbonsäure. Bevorzugt ist hierunter Citronensäure und ihre Salze.

Geeignete organische Builder sind weiterhin Phosphonsäuren, wie z. B. Hydroxyalkylphosphonsäuren, Aminophosphonsäuren und die Salze davon. Dazu zählen z. B. Phosphonobutantricarbonsäure, Aminotris-methylenphosphonsäure, Ethylendiamintetraethylenphosphonsäure, Hexamethylendiamintetramethylenphosphonsäure, Diethylentriaminpentamethylenphosphonsäure, Morpholino-methandiphosphonsäure, 1-Hydroxy-C₁-C₁₀-alkyl-1,1-diphosphonsäuren wie 1-Hydroxyethan-1,1-diphosphonsäure. Bevorzugt ist hierunter 1-Hydroxyethan-1,1-diphosphonsäure und deren Salze.

Geeignete organische Builder sind weiterhin Aminopolycarbonsäuren, wie Nitrilotriessigsäure (NTA), Nitrilomonoessigdipropionsäure, Nitrilotripropionsäure, β-Alanindiessigsäure (β-ADA), Ethylendiamintetraessigsäure (EDTA), Diethylentriaminpentaessigsäure, 1,3-Propylendiamintetraessigsäure, 1,2-Propylendiamintetraessigsäure, N-(Alkyl)-ethylendiamintriessigsäure, N-(Hydroxyalkyl)-ethylendiamintriessigsäure, Ethylendiamintriessigsäure, Cyclohexylen-1,2-diamintetraessigsäure, Iminodibernsteinsäure, Ethylendiamindibernsteinsäure, Serindiessigsäure, Isoserindiessigsäure, L-Asparagindiessigsäure, L-Glutamindiessigsäure, Methylglycindiessigsäure (MGDA) und die Salze der zuvor genannten Aminopolycarbonsäuren. Bevorzugt sind hierunter L-Glutamindiessigsäure, Methylglycindiessigsäure und deren Salze.

Geeignete organische Builder sind weiterhin polymere Carbonsäuregruppen-haltige Verbindungen, wie Acrylsäure-Homopolymere. Diese weisen vorzugsweise ein zahlenmittleres Molekulargewicht im Bereich von 800 bis 70000 g/mol, besonders bevorzugt von 900 bis 50000 g/mol, insbesondere von 1000 bis 20000 g/mol, speziell 1000 bis 10000 g/mol, auf. Der Begriff Acrylsäure-Homopolymer umfasst dabei auch Polymere, in denen die Carbonsäuregruppen teilweise oder vollständig neutralisiert vorliegen. Dazu zählen Acrylsäure-Homopolymere, in denen die Carbonsäuregruppen teilweise oder vollständig in Form von Alkalimetallsalzen oder Ammoniumsalzen vorliegen. Bevorzugt sind Acrylsäure-Homopolymere, in denen die Carbonsäuregruppen protoniert sind oder in denen die Carbonsäuregruppen teilweise oder vollständig in Form von Natriumsalzen vorliegen.

Geeignete polymere Carbonsäuregruppen-haltige Verbindungen sind auch Oligomaleinsäuren, wie sie beispielsweise in EP-A 451 508 und EP-A 396 303 beschrieben sind.

Geeignete polymere Carbonsäuregruppen-haltige Verbindungen sind auch Terpolymere ungesättigter C₄-C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können. Als ungesättigte C₄-C₈-Dicarbonsäuren sind hierbei beispielsweise Maleinsäure, Fumarsäure, Itaconsäure und Citraconsäure geeignet. Bevorzugt wird Maleinsäure. Die Gruppe (i) umfasst monoethylenisch ungesättigte C₃-C₈-Monocarbonsäuren, wie z. B. Acrylsäure, Methacrylsäure, Crotonsäure und Vinylessigsäure. Bevorzugt werden aus der Gruppe (i) Acrylsäure und Methacrylsäure eingesetzt. Die Gruppe (ii) umfasst monoethylenisch ungesättigte C₂-C₂₂-Olefine, Vinylalkylether mit C₁-C₈-Alkylgruppen, Styrol, Vinylester von C₁-C₈-Carbonsäuren, (Meth)acrylamid und Vinylpyrrolidon. Bevorzugt werden aus der Gruppe (ii) C₂-C₆-Olefine, Vinylalkylether mit C₁-C₄-Alkylgruppen, Vinylacetat und Vinylpropionat eingesetzt. Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt. Die Gruppe (iii) umfasst (Meth)acrylester von C₁-C₈-Alkoholen, (Meth)acrylnitril, (Meth)acrylamide von C₁-C₈-Aminen, N-Vinylformamid und N-Vinylimidazol.

Geeignete polymere Carbonsäuregruppen-haltige Verbindungen sind auch Homopolymere der monoethylenisch ungesättigten C₃-C₈-Monocarbonsäuren, wie z. B. Acrylsäure, Methacrylsäure, Crotonsäure und Vinylessigsäure, insbesondere der Acrylsäure und Methacrylsäure, Copolymere von Dicarbonsäuren, wie z. B. Copolymere von Maleinsäure und Acrylsäure im Gewichtsverhältnis 10 : 90 bis 95 : 5, besonders bevorzugt solche im Gewichtsverhältnis 30 : 70 bis 90 : 10 mit Molmassen von 1000 bis 150000; Terpolymere aus Maleinsäure, Acrylsäure und einem Vinylester einer C₁-C₃-Carbonsäure im Gewichtsverhältnis 10 (Maleinsäure) : 90 (Acrylsäure + Vinylester) bis 95 (Maleinsäure) : 10 (Acrylsäure + Vinylester), wobei das Gew.-Verhältnis von Acrylsäure zum Vinylester im Bereich von 30 : 70 bis 70 : 30 variieren kann; Copolymere von Maleinsäure mit C₂-C₈-Olefinen im Molverhältnis 40 : 60 bis 80 : 20, wobei Copolymere von Maleinsäure mit Ethylen, Propylen oder Isobuten im Molverhältnis 50 : 50 besonders bevorzugt sind.

Geeignete polymere Carbonsäuregruppen-haltige Verbindungen sind weiterhin Copolymere von 50 bis 98 Gew.-% ethylenisch ungesättigter schwacher Carbonsäuren mit 2 bis 50 Gew.-% ethylenisch ungesättigter Sulfonsäuren, wie sie beispielsweise in der EP-A-0877002 beschrieben sind. Geeignete schwache ethylenisch ungesättigte Carbonsäuren sind insbesondere C₃-C₆-Monocarbonsäuren, wie Acrylsäure und Methacrylsäure. Geeignete ethylenisch ungesättigte Sulfonsäuren sind 2-Acetylamidomethyl-1-propansulfonsäure, 2-Methacrylsäureamido-2-methyl-1-propansulfonsäure, 2-Methacrylamindo-2-hydroxypropansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen-1-sulfonsäure, Styrolsulfonsäure, Vinylsulfonsäure, 3-Sulfopropylacrylat, 3-Sulfopropylmethacrylat, Sulfomethylacrylamid, Sulfomethylmethacrylamid und Salze dieser Säuren. Die Copolymere können weiterhin 0 bis 30 Gew.-% ethylenisch ungesättigter C₄-C₈-Dicarbonsäuren, wie Maleinsäure, sowie 0 bis 30 Gew.-% wenigstens eines Monomers, das mit den zuvor genannten Monomeren copolymerisierbar ist, einpolymerisiert enthalten. Bei Letzterem handelt es sich beispielsweise um C₁-C₄-Alkylester von (Meth)Acrylsäure, C₁-C₄-Hydroxyalkylester von (Meth)Acrylsäure, Acrylamid, Alkyl-substituiertes Acrylamid, N,N-Dialkyl-substituiertes Acrylamid, Vinylphosphonsäure, Vinylacetat, Allylalkohole, sulfonierte Allylalkohole, Styrol und andere Vinylaromaten, Acrylonitril, N-Vinylpyrrolidon, N-Vinylformamid, N-Vinylimidazol oder N-Vinylpyridin. Das gewichtsmittlere Molekulargewicht dieser Copolymere liegt im Bereich von 3000 bis 50000. Besonders geeignet sind Copolymere mit etwa 77 Gew.-% wenigstens einer ethylenisch ungesättigten C₃-C₆-Monocarbonsäure und etwa 23 Gew.-% wenigstens einer ethylenisch ungesättigten Sulfonsäure.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls. Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden. Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii). Als Pfropfgrundlage sind abgebaute Polysaccharide, wie z. B. sauer oder enzymatisch abgebaute Stärken, Inuline oder Zellulose, Eiweisshydrolysate und reduzierte (hydrierte oder hydrierend aminierte) abgebaute Polysaccharide, wie z. B. Mannit, Sorbit, Aminosorbit und N-Alkylglucamin, geeignet sowie auch Polyalkylenglykole mit Molmassen mit bis zu M_{w} = 5000, wie z. B. Polyethylenglykole, Ethylenoxid/Propylenoxid bzw. Ethylenoxid/Butylenoxid bzw. Ethylenoxid/Propylenoxid/Butylenoxid-Blockcopolymere und alkoxylierte ein- oder mehrwertige C₁-C₂₂-Alkohole.(vgl. US-A-5756456).

Ebenfalls geeignet sind Polyglyoxylsäuren, wie sie beispielsweise in EP-B-001004, US-A-5399286, DE-A-4106355 und EP-A-656914 beschrieben sind. Die Endgruppen der Polyglyoxylsäuren können unterschiedliche Strukturen aufweisen.

Weiterhin sind Polyamidocarbonsäuren und modifizierte Polyamidocarbonsäuren geeignet; diese sind beispielsweise aus EP-A-454126, EP-B-511037, WO-A94/01486 und EP-A-581452 bekannt.

Auch Polyasparaginsäuren oder Cokondensate der Asparaginsäure mit weiteren Aminosäuren, C₄-C₂₅-Mono- oder -Dicarbonsäuren und/oder C₄-C₂₅-Mono- oder - Diaminen können als polymere Carbonsäuregruppen-haltige Verbindungen eingesetzt werden. Besonders bevorzugt werden in phosphorhaltigen Säuren hergestellte, mit C₆-C₂₂-Mono- oder -Dicarbonsäuren bzw. mit C₆-C₂₂-Mono- oder -Diaminen modifizierte Polyasparaginsäuren eingesetzt.

Unter den polymeren Carbonsäuregruppen-haltigen Verbindungen sind Polyacrylsäuren, auch in teilweise oder vollständig neutralisierter Form, bevorzugt.

Als organische Builder eignen sich weiterhin Iminodibernsteinsäure, Oxydibernsteinsäure, Aminopolycarboxylate, Alkylpolyaminocarboxylate, Aminopolyalkylenphosphonate, Polyglutamate, hydrophob modifizierte Citronensäure, wie z. B. Agaricinsäure, Poly-[alpha]-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Builder verwendet werden.

Bevorzugt wird als Komponente b) ein Gemisch verschiedener Builder eingesetzt.

### Komponente c)

Die Enzyme sind vorzugsweise ausgewählt unter Hydrolasen, wie Proteasen, Esterasen, Glucosidasen, Lipasen, Amylasen, Cellulasen, Mannanasen, anderen Glykosylhydrolasen und Gemischen der zuvor genannten Enzyme. Alle diese Hydrolasen tragen zur Schmutzauflösung und -entfernung von protein-, fett- oder stärkehaltigen Verschmutzungen bei. Zur Bleiche können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe.

Geeignete Hydrolasen sind z. B. α-Glucosidasen (EC-Nummer 3.2.1.20), Proteasen (Ovozyme® (von Novozymes); EC-Nummer 3.2.1.20), Amylasen [Purastar® (von Genencor), Termamyl® (von Novozymes), Stainzyme® (von Novozymes), Duramyl® (von Novozymes)], Mannanasen [Purabrite® (von Genencor), Mannastar® (von Genencor), Mannaway® (von Novozymes)] und Cellulasen [Carezyme® (von Novozymes), Celluzyme® (von Novozymes), Endolase, Puradax® (von Genencor)]. Zu den geeigneten Amylasen zählen insbesondere α-Amylasen (EC-Nummer 3.2.1.1), Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Geeignete Lipasen sind Esterasen, wie Lipex und Lipolase. Beispiele für lipolytisch wirkende Enzyme sind die bekannten Cutinasen.

Auch Peroxidasen oder Oxidasen haben sich in einigen Fällen als geeignet erwiesen.

Vorzugsweise enthält das erfindungsgemäße Geschirrspülmittel wenigstens eine Protease und/oder Amylase.

Besonders bevorzugt sind Protease und/oder Amylase-haltige Mischungen. Bevorzugt als Proteasen in den zuvor genannten Mischungen sind Proteasen vom Subtilisin-Typ (Savinase, etc.; EC-Nummer 3.4.21.62).

Die Enzyme können an Trägerstoffe adsorbiert sein, um sie gegen vorzeitige Zersetzung zu schützen.

Gegebenenfalls kann das erfindungsgemäße Geschirrspülmittel noch Enzymstabilisatoren, z. B. Calciumpropionat, Natriumformiat oder Borsäuren oder deren Salze, und/oder Oxidationsverhinderer enthalten.

### Komponente d)

Bei den Bleichmitteln d) handelt es sich vorzugsweise um Bleichsysteme, die neben Bleichmitteln gegebenenfalls noch Bleichaktivatoren, Bleichkatalysatoren und/oder Bleichstabilisatoren enthalten.

Geeignete Bleichmittel sind beispielsweise Percarbonsäuren, z. B. Diperoxododecandicarbonsäure, Phthalimidopercapronsäure oder Monoperoxophthalsäure oder -terephthalsäure, Salze der Percarbonsäuren, z. B. Natriumpercarbonat, Addukte von Wasserstoffperoxid an anorganische Salze, z. B. Natriumperborat-Monohydrat, Natriumperborat-Tetrahydrat, Natriumcarbonat-Perhydrat oder Natriumphosphat-Perhydrat, Addukte von Wasserstoffperoxid an organische Verbindungen, z. B. Harnstoff-Perhydrat, oder von anorganischen Peroxosalzen, z. B. Alkalimetallpersulfaten, oder -peroxodisulfaten.

Als Bleichaktivatoren eignen sich beispielsweise polyacylierte Zucker, z. B. Pentaacetylglucose; Acyloxybenzolsulfonsäuren und deren Alkali- und Erdalkaiimetallsalze, z. B. Natrium-p-nonanoyloxybenzolsulfonat oder Natrium-p-benzoyloxybenzolsulfonat; N,N-diacylierte und N,N,N',N'-tetraacylierte Amine, z. B. N,N,N',N'-Tetraacetylmethylendiamin und -ethylendiamin (TAED), N,N-Diacetylanilin, N,N-Diacetyl-ptoluidin oder 1,3-diacylierte Hydantoine, wie 1,3-Diacetyl-5,5-dimethylhydantoin; N-Alkyl-N-sulfonylcarbonamide, z. B. N-Methyl-N-mesylacetamid oder N-Methyl-N-mesylbenzamid; N-acylierte cyclische Hydrazide, acylierte Triazole oder Urazole, z. B. Monoacetylmaleinsäurehydrazid; O,N,N-trisubstituierte Hydroxylamine, z. B. O-Benzoyl-N,N-succinylhydroxylamin, O-Acetyl-N,N-succinylhydroxylamin oder O,N,N-Triacetylhydroxylamin; N,N'-Diacylsulfurylamide, z. B. N,N'-Dimethyl-N,N'-diacetylsulfurylamid oder N,N'-Diethyl-N,N'-dipropionylsulfurylamid; acylierte Lactame, wie beispielsweise Acetylcaprolactam, Octanoylcaprolactam, Benzoylcaprolactam oder Carbonylbiscaprolactam; Anthranilderivate, wie z. B. 2-Methylanthranil oder 2-Phenylanthranil; Triacylcyanurate, z. B. Triacetylcyanurat oder Tribenzoylcyanurat; Oximester und Bisoximester, wie z. B. O-Acetylacetonoxim oder Bisisopropyliminocarbonat; Carbonsäureanhydride, z. B. Essigsäureanhydrid, Benzoesäureanhydrid, m-Chlorbenzoesäureanhydrid oder Phthalsäureanhydrid; Enolester, wie z. B. Isopropenylacetat; 1,3-Diacyl-4,5-diacyloxyimidazoline, z. B. 1,3-Diacetyl-4,5-diacetoxyimidazolin; Tetraacetylglycoluril und Tetrapropionylglycoluril; diacylierte 2,5-Diketopiperazine, z. B. 1,4-Diacetyl-2,5-diketopiperazin; ammoniumsubstituierte Nitrile, wie z. B. N-Methylmorpholiniumacetonitrilmethylsulfat; Acylierungsprodukte von Propylendiharnstoff und 2,2-Dimethyl-propylendihamstoff, z. B. Tetraacetylpropylendihamstoff; α-Acyloxypolyacylmalonamide, z. B. α-Acetoxy-N,N'-diacetylmalonamid; Diacyldioxohexahydro-1,3,5-triazine, z. B. 1,5-Diacetyl-2,4-dioxohexahydro1,3,5-triazin; Benz-(4H)1,3-oxazin-4-one mit Alkylresten, z. B. Methyl, oder aromatischen Resten, z. B. Phenyl, in der 2-Position.

Ein Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise in US-A 5 360 569 und EP-A 453 003 beschrieben sind. Besonders wirksame Bleichkatalysatoren sind Mangankomplexe, die beispielsweise in der WO-A 94/21777 beschrieben sind. Solche Verbindungen werden im Falle ihres Einsatzes in den Wasch- und Reinigungsmitteln höchstens in Mengen bis 1,5 Gew.-%, insbesondere bis 0,5 Gew.-%, im Falle von sehr aktiven Mangankomplexen in Mengen bis zu 0,1 Gew.-%, eingearbeitet. Neben dem beschriebenen Bleichsystem aus Bleichmitteln, Bleichaktivatoren und gegebenenfalls Bleichkatalysatoren ist für die erfindungsgemäßen Wasch- und Reinigungsmittel auch die Verwendung von Systemen mit enzymatischer Peroxidfreisetzung oder von photoaktivierten Bleichsystemen möglich.

### Komponente f)

Die erfindungsgemäß verwendeten Polymerzusammensetzungen eignen sich alleine bereits zur Modifizierung der rheologischen Eigenschaften im Sinne einer Verdickung.

Um der erfindungsgemäß verwendeten Formulierung für die maschinelle Geschirrreinigung die erwünschte Viskosität zu verleihen, kann zusätzlich wenigstens ein Verdicker f) eingesetzt werden.

Geeignet sind grundsätzlich jedwede bekannte Verdicker (Rheologiemodifizierungsmittel), sofern sie keinen negativen Einfluss auf die Wirkung des Geschirrspülmittels ausüben. Geeignete Verdicker können sowohl natürlichen Ursprungs als auch synthetischer Natur sein.

Beispiele für Verdicker natürlichen Ursprungs sind Xanthan, Johannisbrotkernmehl, Guarmehl, Carrageen, Agar, Tragant, Gummi arabicum, Alginate, modifizierte Stärken, wie Hydroxyethylstärke, Stärkephosphatester oder Stärkeacetate, Dextrine, Pektine und Cellulosederivate, wie Carboxymethylcellulose, Hydroxyethylcellulose, Hydroxypropylcellulose, Hydroxypropylmethylcellulose, Methylcellulose und dergleichen.

Verdicker natürlichen Ursprungs sind auch anorganische Verdicker, wie Polykieselsäuren und Tonmineralien, z. B. Schichtsilikate, wie auch die bei den Buildern genannten Silikate.

Beispiele für synthetische Verdicker sind Polyacryl- und Polymethacrylverbindungen, wie (teil)vernetzte Homopolymere der Acrylsäure, beispielsweise mit einem Allylether von Saccharose oder Pentaerythrit oder mit Propylen vernetzte Homopolymere der Acrylsäure (Carbomer), z. B. die Carbopol®-Marken von BF Goodridge (z. B. Carbopol® 676, 940, 941, 934 und dergleichen) oder die Polygel®-Marken von 3V Sigma (z. B. Polygel® DA), Copolymere ethylenisch ungesättigter Mono- oder Dicarbonsäuren, beispielsweise Terpolymere von Acrylsäure, Methacrylsäure oder Maleinsäure mit Methyl- oder Ethylacrylat und einem (Meth)Acrylat, das sich von langkettigen ethoxylierten Alkoholen ableitet, beispielsweise die Acusol®-Marken von Rohm & Haas (z. B. Acusol® 820 oder 1206A), Copolymere von zwei oder mehr Monomeren, die ausgewählt sind unter Acrylsäure, Methacrylsäure und ihren C₁-C₄-Alkylestern, z. B. Copolymere von Methacrylsäure, Butylacrylat und Methylmethacrylat oder von Butylacrylat und Methylmethacrylat, z. B. die Aculyn®- und Acusol®-Marken von Rohm & Haas (z. B. Aculyn® 22, 28 oder 33 und Acusol® 810, 823 und 830), oder vernetzte hochmolekulare Acrylsäurecopolymere, beispielsweise mit einem Allylether von Saccharose oder Pentaerythrit vernetzte Copolymere von C₁₀-C₃₀-Alkylacrylaten mit einem oder mehreren Comonomeren, die ausgewählt sind unter Acrylsäure, Methacrylsäure und ihren C₁-C₄-Alkylestern (z. B. Carbopol® ETD 2623, Carbopol® 1382 oder Carbopol® AQUA 30 von Rohm & Haas).

Beispiele für synthetische Verdicker sind weiterhin Umsetzungsprodukte von Maleinsäurepolymeren mit ethoxylierten langkettigen Alkoholen, z. B. die Surfonic L-Serie von Texaco Chemical Co. oder Gantrez AN-119 von ISP; Polyethylenglykole, Polyamide, Polyimine und Polycarbonsäuren.

Geeignet sind auch Gemische der o. g. Verdicker.

Bevorzugte Verdicker sind Xanthane und die oben genannten Polyacryl- und Polymethacrylverbindungen.

### Komponente g)

Geeignete zusätzliche Tenside g), die von Komponente a) verschieden sind, sind anionische Tenside, nichtionische Tenside, kationische Tenside, amphotere Tenside und Mischungen davon.

Typische Beispiele für anionische Tenside sind Seifen, Alkylsulfonate, Alkylbenzolsulfonate, Olefinsulfonate, Methylestersulfonate, Sulfofettsäuren, Alkylsulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkylglucosecarboxylate, Proteinfettsäurekondensate und Alkyl(ether)phosphate.

Zu weiteren zusätzlichen nichtionischen Tensiden gehören beispielsweise:
- Glycerinester, wie beispielsweise Glycerinmonostearat,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate. Beispielsweise können Cocodimethylsulfopropylbetain, Laurylbetain, Cocamidopropylbetain, Natriumcocamphopropionat oder Tetradecyldimethylaminoxid eingesetzt werden.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide. Beispielsweise können Behenyl oder Cetyltrimethylammoniumchlorid eingesetzt werden.

Geeignete organische Lösungsmittel g) sind ausgewählt unter ein- oder mehrwertigen Alkoholen, Alkanolaminen oder Glykolethern. Vorzugsweise sind sie ausgewählt unter Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propylether, Dipropylenglykolmonomethyl- oder -ethylether, Diisopropylenglykolmonomethyl- oder -ethylether, Methoxy-, Ethoxy- oder Butoxytriglykol, i-Butoxyethoxy-2-propanol, 3-Methyl-3-methoxybutanol, Propylen-glykol-t-butylether sowie Mischungen dieser Lösemittel.

Als Schauminhibitoren oder Entschäumer g) kommen beispielsweise Seifen, Paraffine oder Silikonöle in Betracht, die gegebenenfalls auf Trägermaterialien aufgebracht sein können.

Geeignete Basen g) sind auf Alkalimetallhydroxide, Erdalkalimetallhydroxide, Alkalimetallcarbonate, Erdalkalimetallcarbonate, Ammoniumcarbonat Alkalimetallhydrogencarbonate, Erdalkalimetallhydrogencarbonate, Ammoniumhydrogencarbonat und Mischungen davon. Vorzugsweise werden Na-, Li- und Mg-Carbonate bzw. -Hydrogencarbonate, insbesondere Natriumcarbonat und/oder Natriumhydrogencarbonat, eingesetzt.

Gegenstand der vorliegenden Anmeldung ist weiterhin ein Verfahren zur Reinigung von Geschirr in einer Geschirrspülmaschine, unter Einsatz erfindungsgemäßer Formulierungen, die die Polymerzusammensetzung a) enthalten, für die maschinelle Geschirrreinigung, wobei die Formulierungen vorzugsweise während des Durchlaufens eines Geschirrspülprogramms, vor Beginn des Hauptspülgangs oder im Verlaufe des Hauptspülgangs in den Innenraum einer Geschirrspülmaschine eindosiert werden. Die Eindosierung bzw. der Eintrag der erfindungsgemäß verwendeten Formulierungen in den Innenraum der Geschirrspülmaschine kann manuell erfolgen, vorzugsweise wird das Mittel jedoch mittels einer Dosierkammer der Geschirrspülmaschine in den Innenraum der Geschirrspülmaschine dosiert. Die erfindungsgemäß verwendeten Formulierungen zeigen ihre vorteilhaften Reinigungseigenschaften insbesondere auch in Niedrigtemperatur-Reinigungsverfahren. Diese Verfahren werden bei Temperaturen bis maximal 55 °C, vorzugsweise bis maximal 50 °C, durchgeführt. Die erfindungsgemäßen Formulierungen zeichnen sich gegenüber herkömmlichen Formulierungen für die maschinelle Geschirrreinigung durch eine gute Reinigungsleistung aus. Sie eignen sich weiterhin zur Verbesserung der Trocknung beim maschinellen Geschirrspülen.

Die Erfindung wird anhand der im Folgenden beschriebenen Figuren und der Beispiele näher erläutert. Dabei sollen die Figuren und Beispiele nicht als einschränkend für die Erfindung verstanden werden.

In den nachfolgenden Figuren und Beispielen werden folgende Abkürzungen verwendet:
EO: Ethylenoxid
PO: Propylenoxid
BO: Butylenoxid
Mₙ: Zahlenmittleres Molekulargewicht
M_{w}: Gewichtsmittleres Molekulargewicht
PET: Polyethylenterephthalat
T_{L}-Wert: Wert für die Lichtdurchlässigkeit
n.b.: nicht bestimmt
rad/s: Radiant pro Sekunde (radiant per second)
pphm: Gewichtsteile pro 100 Gewichtsteile Monomer (parts per hundred monomer).

### FIGURENBESCHREIBUNG

Figur 1 zeigt für das Gel aus Beispiel 8 der Tabelle 1 die Viskosität in Abhängigkeit von der Temperatur.
Figur 2 zeigt für das Gel aus Beispiel 8 der Tabelle 1 das Speichermodul in Abhängigkeit von der Temperatur.
Figur 3 zeigt für das Gel aus Beispiel 8 der Tabelle 1 das Verlustmodul in Abhängigkeit von der Temperatur.
Figur 4 zeigt für das Gel aus Beispiel 8 der Tabelle 1 den Phasenwinkel **δ** in Abhängigkeit von der Temperatur.

### BEISPIELE

### I) Analytik:

### I.a) Bestimmung der Wasserlöslichkeit

Zur Bestimmung der Wasserlöslichkeit wurden 5 g der jeweiligen Polymerzusammensetzung in ein 1 L Becherglas gegeben und 900 mL Wasser, welches zuvor auf 40 °C erhitzt wurde, zugegeben. Die Mischung wurde 20 Minuten lang mit einem Magnetrührer bei 40 °C gerührt und der pH-Wert mit Natronlauge auf 8 eingestellt. Wasserlösliche Polymergele führten zu transparenten oder leicht trüben Lösungen.

### I.b) Bestimmung des gewichtsgemittelten Molekulargewichts (M_{w}):

Das gewichtsgemittelte Molekulargewicht des Polymers wurde durch Gelpermeationschromatographie (GPC) bestimmt. Dazu wurden die folgenden Geräte und Chromatographiemethoden verwendet:
Standard: Polyacrylsäure, neutralisiert
Elutionsmittel: 0,08 Mol/L Tris, pH 7,0, + 0,15 Mol/L Na Cl + 0,01 Mol/L NaN₃ in deionisiertem Wasser
Fluss: 0,8 mL/min
Säulensatz: 1 Vorsäule (I = 5 cm), 2Trennsäulen (I = je 30 cm)
Säulentemperatur: 35 °C
Detektor: DRI (Refractive Index Detector) Agilent 1100

### I.c) Bestimmung der Transparenz:

Die Transparenz der Probekörper wurde anhand ihrer Lichtduchlässigkeit (T_{L}-Wert) bei einer Wellenlänge von 500 nm und bei 20 °C bestimmt. Als 100%-Referenz wurde Wasser verwendet.

### II) Herstellungsbeispiele:

### Allgemeine Herstellungsvorschrift:

In einem Glasreaktor, ausgestattet mit drei Zuläufen, Stickstoffeinlass und einem Ankerrührer, wurde die Polyetherkomponente (PE), gegebenenfalls Regler (R) und gegebenenfalls das Lösungsmittel (LM) in einer Menge gemäß Tabelle 1 vorgelegt, für ein paar Minuten mit Stickstoff gespült und auf 75 °C erwärmt. Zur Vorlage wurden anschließend bei 75 °C und unter Rühren bei 100 Umdrehungen/Minute innerhalb von 4 Stunden die Zuläufe 1 bis 3 simultan zugegeben. Zulauf 1 enthielt Monomer (M), Zulauf 2 enthielt einen Starter (S), gelöst in einer kleinen Menge nicht-ionischem Tensid (PE) und/oder Lösungsmittel (LM), und der Zulauf 3 enthielt gegebenenfalls eine weitere Menge Regler (R). Nach der Zugabe der Zuläufe 1, 2 und 3 wurde die Mischung zur Nachpolymerisation für eine weitere Stunde bei 75 °C und bei 100 Umdrehungen/Minute gerührt. Anschließend wurde das Polymerisat in ein Becherglas gegossen und sofort auf Raumtemperatur abgekühlt.

Zur Herstellung der erfindungsgemäßen Polymerzusammensetzungen wurden folgende Einsatzstoffe verwendet:
- M1:: Acrylsäure

- PE1:: flüssiges nicht-ionisches Tensid; Reaktionsprodukt aus 5,5 Mol EO, 2 Mol PO und 1 Mol Tridecanol
- PE2:: flüssiges nicht-ionisches Tensid; Reaktionsprodukt aus 7,8 Mol EO, 1,2 Mol BO und 1,0 Mol eines mehrheitlich unverzweigten C₉-C₁₁-Oxoalkohols
- PE3:: flüssiger Polyether bestehend aus Polyethylenglycol mit einer mittleren Molmasse von 400 g/mol
- PE4:: festes nicht-ionisches Tensid bestehend aus einem modifizierten Fettalkoholpolyglycolether
- PE5:: C₁₃C₁₅-Alkohol + 9 EO + 2 BuO
- PE6:: C₆-Alkohol + 6 EO (ausgehend von Hexylglycol bzw. Hexyldiglycol)
- PE7:: C₁₃C₁₅-Oxoalkohol + 8 EO + 3,8 PO

- R1:: (2-Ethylhexyl)-thioglycolat
- R2:: 2-Mercaptoethanol
- R3:: NaH₂PO₂ (55%ige wässrige Lösung),

- LM1:: Propylenglycol
- LM2:: Dipropylenglycol
- LM3:: Wasser
- LM4:: Isopropanol

- S1:: tert-Butyl peroxyneodekanoat (Reinheit: 97 %) (CAS-Nr. 26748-41-4)
- S2:: tert-Butyl peroxypivalat (Reinheit: 75 %) (CAS-Nr. 927-07-1)
- S3:: 2,2'-Azobis(2-methylpropionamidine)dihydrochloride (CAS-Nr. 2997-92-4)

Die Herstellung der erfindungsgemäßen Polymerzusammensetzungen der Beispiele 1 bis 13 erfolgte nach der oben beschriebenen allgemeinen Herstellungsvorschrift. Als Monomerkomponente (M1) wurde Acrylsäure verwendet. Die eingesetzte Acrylsäure und die jeweilige Polyetherkomponente (PE) wurden in den in Tabelle 1 angegebenen Gewichtsteilen zugegeben, wobei die Menge des Lösungsmittels (LM), des Starters (S) sowie des Reglers (R) in den Polymermischungen variiert wurden. Die Konsistenz und die analytischen Parameter der daraus resultierenden Polymerzusammensetzungen sind in der nachfolgenden Tabelle 2 zusammengefasst.

**Tabelle 1**

| **Bsp. -Nr.** | **Vorlage** | | | **Zulauf 1** | **Zulauf 2** | | | **Zulauf 3** | |
|---|---|---|---|---|---|---|---|---|---|
| | **PE [g] (PE-Nr.)** | **R [g] (R-Nr.)** | **LM [g] (LM-Nr.)** | **M [g]** | **S [g] (S-Nr.)** | **PE [g] (PE Nr.)** | **LM [g] (LM-Nr.)** | **R [g]** | **LM [g] (LM-Nr.)** |
| 1 | 230 (1) | - | 290(2) | 540 (1) | 5.11 (2) | 10 (1) | 65 (2) | 10.8 (2) + 29.5 (3) | 19.8 (2) |
| 2 | 123 (2) | - | 95 (2) | 253 (1) | 2.40 (2) | 5 (2) | 95 (2) | 51 (1) | - |
| 3 | 123 (5) | 45 (1) | 95 (2) | 253 (1) | 2.40(2) | 5 (5) | 95 (2) | 12 (1) | - |
| 4 | 123 (5) | 43 (1) | 95 (1) | 253 (1) | 2.40(2) | 5 (5) | 95 (1) | 14 (1) | - |
| 5 | 123 (5) | 45 (1) | 95 (1) | 253 (1) | 2.40(2) | 5 (5) | 95 (1) | 12 (1) | - |
| 6 | 123 (2) | 51 (1) | 95 (1) | 253 (1) | 5.93 (1) | 5 (2) | 95 (1) | 6 (1) | - |
| 7 | 123 (7) | 28 (1) | 80 (2) | 285 (1) | 6.68 (1) | 5 (8) | 77 (2) | 29 (1) | - |
| 8 | 230 (5) | - | 290 (2) | 540 (1) | 5.11 (2) | 10 (5) | 65 (2) | 10.8 (2) + 29.5 (3) | 19.8 (2) |
| 9 | 230 (5) | - | 310(2) | 420 (1) | 3.98(2) | 10 (5) | 146 (2) | 8.4 (2) + 22.9 (3) | 48.7 (2) |
| 10 | 250 (3) | - | 247 (3) | 500 (1) | 3.55 (3) | - | 46 (3) | 10.0 (2) + 27.3 (3) | 27.7 (3) |
| 11 | 170 (5) + 60 (6) | - | 320 (2) | 480 (1) | 4.54 (2) | 10 (5) | 75 (2) | 9.6 (2) + 26.2 (3) | 44.2 (2) |
| 12 | 230 (4) | - | 340 (2) | 480 (1) | 4.54 (2) | 10 (4) | 65 (2) | 9.6 (2) + 26.2 (3) | 34.2 (2) |
| 13 | 170 (4) + 60 (6) | - | 341 (2) | 480 (1) | 4.54(2) | 10 (4) | 64 (2) | 9.6 (2) + 26.2 (3) | 34.2(2) |

**Tabelle 2**

| **Bsp.-Nr.** | **T_{L}-Wert bei 500 nm** | **Konsistenz bei 25 °C** | **Mw (g/mol)** |
|---|---|---|---|
| 1 | n.b. | fest | 4700 |
| 2 | n.b. | fest | 1700 |
| 3 | 99.3% | fest | 6000 |
| 4 | 98.3% | fest | 2200 |
| 5 | 97.9% | fest | 7100 |
| 6 | 97.4% | fließend | 9500 |
| 7 | n.b. | fest | 4300 |
| 8 | 98.7% | fest | 4200 |
| 9 | 99.7% | fließend | 4200 |
| 10 | 99.1% | fließend | 5800 |
| 11 | 99.0% | fest | 4200 |
| 12 | 99.6% | fließend | 4500 |
| 13 | 99.7% | fließend | 6900 |

### III) Bestimmung der stofflichen Eigenschaften:

### III.a) Viskoelastische Parameter

### Viskosität in Abhängigkeit der Temperatur:

Der Viskositätsverlauf in Abhängigkeit der Temperatur der zu untersuchenden Proben wurde mittels eines Rotationsrheometers (DHR-1 der Firma TA-Instruments mit Peltiersystem, Platte/Platte-Geometrie, Ø 40 mm, h =1 mm) bei Temperaturen von 20 °C bis 80 °C untersucht. Temperatur-Rampe (γ = 1 % mit *M*ₘᵢₙ = 100 µNm). Messtemperatur(en) von 80 °C zu 20 °C und zurück je zwei Läufe (Kühl-/Heizrate 2 K/min). Messzeit je 30 min.

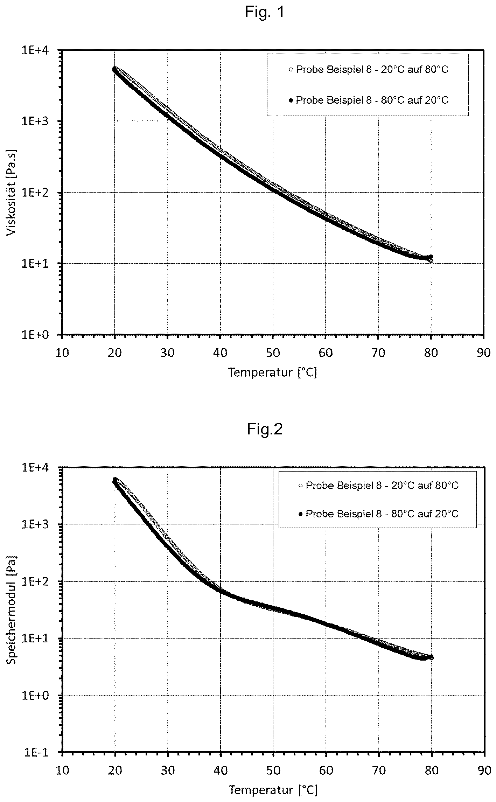

Zur Untersuchung des viskoelastischen Verhaltens der erfindungsgemäßen Polymerzusammensetzungen wurden die Viskositätsverläufe der Polymerzusammensetzungen aus dem Herstellungsbeispiel 8 in Abhängigkeit der Temperatur bestimmt. Die Resultate sind in Figuren 1 bis 4 dargestellt. Wie aus den Diagrammen ersichtlich, der Verlauf der Viskosität (Figur 1) liegt bei den Auf- und Abwärtskurven nicht deckungsgleich aufeinander, was auf die Differenz zwischen der vorgegebenen und der tatsächlichen Temperatur der Probe zurückzuführen ist. Es wurden sowohl für die Abwärts- (80 °C zu 20 °C) als auch für die Aufwärts- (20 °C zu 80 °C) Messungen je zwei Läufe durchgeführt (run 1 und run 2). Hierbei zeigt sich, dass sich die Probe nicht verändert hat.

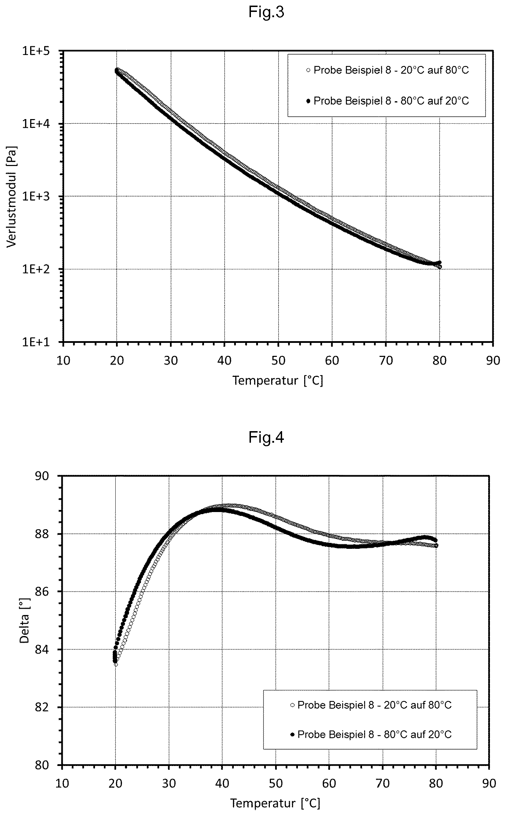

Der Verlauf des Speichermoduls wurde in Figur 2 und der Verlauf des Verlustmoduls wurde in Figur 3 überlagert. Generell ist im Bereich von 35 °C bis 45 °C ein noch näher zu betrachtender Übergang zu beobachten, der sich insbesondere im Maximum des in Figur 4 dargestellten Phasenwinkels **δ** bemerkbar macht.

### Beispiele für Geschirrreinigerformulierungen

### Gesamtgewicht: 10 bis 25 g

| | Phosphatfreie Formulierungen | Phosphatbasierte Formulierungen |
|---|---|---|
| Menge | Menge [Gew.-%] | Menge [Gew.-%] |
| Protease | 1-4 | 1-4 |
| Amylase | 0,2-3 | 0,2-3 |
| Polymerzusammensetzung (Nr. 1-33) | 2-30 | 2-30 |
| Na-Percarbonat | 5-14 | 5-14 |
| TAED (100 %) | 2-8 | 2-8 |
| Na-Disilikat | 1-8 | 1-8 |
| Natriumcarbonat | 5-40 | 5-40 |
| Na-Citrat dihydrat | 0-45 | 1-10 |
| MGDA | 0-45 | 0-20 |
| Tripolyphosphat | 0 | 10-60 |
| Polyvinlyalkohol (Folie) | 0-15 | 0-15 |
| HEDP | 0,5-1,5 | 0,5-1,5 |
| Weitere Inhaltsstoffe (z. B. Parfum, Korrosionsinhibitoren, Tablettierhilfsmittel, Farbstoffe, weitere Hilfsmittel) | | |
| Insgesamt | 100 | 100 |

Die Polymerzusammensetzung kann in die Formulierung eingearbeitet werden oder in der Gesamtformulierung in einem separaten Bereich als klare Gelkomponente von den übrigen Komponenten separat vorliegen (z. B. abgetrennt durch eine Polyvinylalkohol-Folie).

## Patentansprüche

1. Verwendung einer gelförmigen Polymerzusammensetzung, erhältlich durch ein Verfahren, bei dem man
a) eine Monomerzusammensetzung M) bereitstellt, die aus
A) wenigstens einer α,β-ethylenisch ungesättigten Säure besteht und die keine vernetzend wirkenden Monomere B) enthält, die zwei oder mehr als zwei polymerisierbare α,β-ethylenisch ungesättigte Doppelbindungen pro Molekül aufweisen,
b) die in Schritt a) bereitgestellte Monomerzusammensetzung M) einer radikalischen Polymerisation in Gegenwart wenigstens einer Polyetherkomponente PE) unterzieht, die ausgewählt ist unter Polyetherolen mit einem zahlenmittleren Molekulargewicht von wenigstens 200 g/mol und deren Mono- und Di-(C₁-C₆-alkylethern), Polyethergruppen-haltigen Tensiden und Mischungen davon,
in Formulierungen für die maschinelle Geschirrreinigung.

2. Verwendung nach Anspruch 1, wobei die radikalische Polymerisation in Schritt b) zusätzlich in Gegenwart eines Lösungsmittels LM) erfolgt, das ausgewählt ist unter Wasser, C₁-C₆-Alkanolen, von PE) verschiedenen Polyolen, deren Mono- und Dialkylethern und Mischungen davon.

3. Verwendung nach einem der vorhergehenden Ansprüche, wobei die Polymerisation in Schritt b) in Zulauf-Fahrweise erfolgt, wobei man wenigstens einen Teil der Polyetherkomponente PE) und gegebenenfalls, falls vorhanden, wenigstens einen Teil des Lösungsmittels LM) vorlegt, und wenigstens einen Teil der in Schritt a) bereitgestellten Monomerzusammensetzung und wenigstens einen Radikalstarter S) der Vorlage zuführt.

4. Verwendung nach einem der Ansprüche 2 oder 3, wobei die radikalische Polymerisation in Schritt b) zusätzlich in Gegenwart eines Lösungsmittels LM) erfolgt und
- das Reaktionsgemisch zu Beginn der Reaktion in Schritt b) das Lösungsmittel LM) in einer Menge von 0 bis 90 Gew.-%, bevorzugt 0,1 bis 70 Gew.-%, bezogen auf das Gesamtgewicht des Reaktionsgemischs enthält,
- das Reaktionsgemisch nach Abschluss der Reaktion in Schritt b) das Lösungsmittel LM) in einer Menge von 0 bis 50 Gew.-%, bevorzugt 0,1 bis 40 Gew.-%, bezogen auf das Gesamtgewicht des Reaktionsgemischs enthält.

5. Verwendung nach einem der vorhergehenden Ansprüche, wobei die in Schritt a) bereitgestellte Monomerzusammensetzung aus Acrylsäure A1) und gegebenenfalls wenigstens einer weiteren α,β-ethylenisch ungesättigten Säure A2) besteht, die ausgewählt ist unter Carbonsäuren, Sulfonsäuren, Phosphonsäuren und Mischungen davon.

6. Verwendung nach einem der Ansprüche 1 bis 5, wobei die Monomerzusammensetzung M) ausschließlich aus Acrylsäure besteht.

7. Verwendung nach einem der vorhergehenden Ansprüche, wobei die Polyetherkomponente PE) wenigstens ein Polyetherol mit einem zahlenmittleren Molekulargewicht im Bereich von etwa 200 bis 100000 oder einen Mono- oder Di-(C₁-C₂-alkylether) davon umfasst.

8. Verwendung nach einem der vorhergehenden Ansprüche, wobei die Polyetherkomponente PE) wenigstens ein Polyethergruppen-haltiges Tensid umfasst, ausgewählt unter Alkylpolyoxyalkylenethern, Arylpolyoxyalkylenethern, Alkylarylpolyoxyalkylenethern, alkoxylierten tierischen und/oder pflanzlichen Fette und/oder Ölen, Fettaminalkoxylaten, Fettsäureamidalkoxylaten, Fettsäurediethanolamidalkoxylaten, Polyoxyethylensorbitanfettsäureestern, Alkylpolyethersulfaten, Arylpolyethersulfaten, Alkylarylpolyethersulfaten, Alkylpolyethersulfonaten, Arylpolyethersulfonaten, Alkylarylpolyethersulfonaten, Alkylpolyetherphosphaten, Arylpolyetherphosphaten, Alkylarylpolyetherphosphaten, Glycerinethersulfonaten, Glycerinethersulfaten, Monoglycerid(ether)sulfaten, Fettsäureamidethersulfaten, Polyoxyalkylensorbitanfettsäureestern und Mischungen davon.

9. Verwendung nach einem der vorhergehenden Ansprüche, wobei die Polymerzusammensetzung einen Gehalt an Säuregruppen von 1.5 mmol/g bis 15 mmol/g, bevorzugt 4 mmol/g bis 7 mmol/g, aufweist.

10. Verwendung nach einem der vorhergehenden Ansprüche, wobei die Polymerzusammensetzung eine Lichtdurchlässigkeit, angegeben als T_{L}-Wert, gemessen bei 500 nm, von mindestens 85 %, besonders bevorzugt von mindestens 90 %, bezogen auf die Lichtdurchlässigkeit von Wasser, aufweist.

11. Verwendung nach einem der Ansprüche 1 bis 10, wobei in der Polymerzusammensetzung bis zu 80 mol% der Säuregruppen, bevorzugt bis zu 70 mol% der Säuregruppen, insbesondere bis zu 20 mol% der Säuregruppen neutralisiert vorliegen.

12. Verwendung nach einem der Ansprüche 1 bis 10, wobei in der Polymerzusammensetzung die Säuregruppen nicht neutralisiert vorliegen.

13. Verwendung nach einem der vorhergehenden Ansprüche, wobei die in der Polymerzusammensetzung enthaltenen Polymere ein gewichtsmittleres Molekulargewicht, bestimmt mittels Gelpermeationschromatographie (GPC) unter Verwendung von neutralisierter Polyacrylsäure als Polymerstandard, von 1000 bis 70000 Dalton aufweisen.

14. Verwendung nach einem der vorhergehenden Ansprüche, wobei die Formulierung für die maschinelle Geschirrreinigung folgende Komponenten umfasst:
a) wenigstens eine Polymerzusammensetzung, wie in einem der Ansprüche 1 bis 13 definiert,
b) wenigstens einen Builder,
c) gegebenenfalls wenigstens ein Enzym,
d) gegebenenfalls wenigstens ein Bleichmittel,
e) Wasser,
f) gegebenenfalls wenigstens einen Verdicker, und
g) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Löslichkeitsvermittlern und organischen Lösungsmitteln.

15. Verfahren zur maschinellen Reinigung von Geschirr, bei dem man das zu reinigende Geschirr mit einer Behandlungslösung in Kontakt bringt, die eine Formulierungen für die maschinelle Geschirrreinigung, wie in Anspruch 14 definiert, enthält.

## Claims

1. The use of a polymer composition in gel form, obtainable by a process in which
a) a monomer composition M) is provided, consisting of
A) at least one α, β-ethylenically unsaturated acid, and which does not comprise any crosslinking monomers B) having two or more than two polymerizable α,β-ethylenically unsaturated double bonds per molecule,
b) the monomer composition M) provided in step a) is subjected to a free-radical polymerization in the presence of at least one polyether component PE) selected from polyetherols having a number-average molecular weight of at least 200 g/mol and the mono- and di- (C₁-C₆-alkyl ethers) thereof, surfactants containing polyether groups and mixtures thereof,
in formulations for machine dishwashing.

2. The use according to claim 1,wherein the free-radical polymerization in step b) is additionally effected in the presence of a solvent S) selected from water, C₁-C₆-alkanols, polyols other than PE), the mono- and dialkyl ethers thereof, and mixtures thereof.

3. The use according to any of the preceding claims, wherein the polymerization in step b) is effected in feed mode, by initially charging at least a portion of the polyether component PE) and optionally, if present, at least a portion of the solvent S), and supplying at least a portion of the monomer composition provided in step a) and at least one free-radical initiator FRI) to the initial charge.

4. The use according to either of claims 2 and 3, wherein the free-radical polymerization in step b) is additionally effected in the presence of a solvent S) and
- the reaction mixture at the start of the reaction in step b) comprises the solvent S) in an amount of 0% to 90% by weight, preferably 0.1% to 70% by weight, based on the total weight of the reaction mixture,
- the reaction mixture after conclusion of the reaction in step b) comprises the solvent S) in an amount of 0% to 50% by weight, preferably 0.1% to 40% by weight, based on the total weight of the reaction mixture.

5. The use according to any of the preceding claims, wherein the monomer composition provided in step a) consist of acrylic acid A1) and optionally at least one further α,β-ethylenically unsaturated acid A2) selected from carboxylic acids, sulfonic acids, phosphoric acids and mixtures thereof.

6. The use according to any of claims 1 to 5, wherein the monomer composition M) consists exclusively of acrylic acid.

7. The use according to any of the preceding claims, wherein the polyether component PE) comprises at least one polyetherol having a number-average molecular weight in the range from about 200 to 100 000 or a mono- or di-(C₁-C₂-alkyl ether) thereof.

8. The use according to any of the preceding claims, wherein the polyether component PE) comprises at least one surfactant containing polyether groups, selected from alkyl polyoxyalkylene ethers, aryl polyoxyalkylene ethers, alkylaryl polyoxyalkylene ethers, alkoxylated animal and/or vegetable fats and/or oils, fatty amine alkoxylates, fatty acid amide alkoxylates, fatty acid diethanolamide alkoxylates, polyoxyethylene sorbitan fatty acid esters, alkyl polyether sulfates, aryl polyether sulfates, alkylaryl polyether sulfates, alkyl polyether sulfonates, aryl polyether sulfonates, alkylaryl polyether sulfonates, alkyl polyether phosphates, aryl polyether phosphates, alkylaryl polyether phosphates, glyceryl ether sulfonates, glyceryl ether sulfates, monoglyceride (ether) sulfates, fatty acid amide ether sulfates, polyoxyalkylene sorbitan fatty acid esters and mixtures thereof.

9. The use according to any of the preceding claims, wherein the polymer composition has a content of acid groups of 1.5 mmol/g to 15 mmol/g, preferably 4 mmol/g to 7 mmol/g.

10. The use according to any of the preceding claims, wherein the polymer composition has a transparency, reported as the T_{L} measured at 500 nm, of at least 85%, more preferably of at least 90%, based on the transparency of water.

11. The use according to any of claims 1 to 10, wherein up to 80 mol% of the acid groups, preferably up to 70 mol% of the acid groups, especially up to 20 mol% of the acid groups, in the polymer composition are in neutralized form.

12. The use according to any of claims 1 to 10, wherein the acid groups in the polymer composition are not in neutralized form.

13. The use according to any of the preceding claims, wherein the polymers present in the polymer composition have a weight-average molecular weight, determined by means of gel permeation chromatography (GPC) using neutralized polyacrylic acid as polymer standard, of 1000 to 70 000 daltons.

14. The use according to any of the preceding claims, wherein the formulation for machine dishwashing comprises the following components:
a) at least one polymer composition as defined in any of claims 1 to 13,
b) at least one builder,
c) optionally at least one enzyme,
d) optionally at least one bleach,
e) water,
f) optionally at least one thickener, and
g) optionally at least one additive, preferably selected from the following additives other than a) : surfactants, bases, corrosion inhibitors, defoamers, dyes, fragrances, fillers, solubilizers and organic solvents.

15. A method for machine cleaning of dishware, in which the dishware to be cleaned is contacted with a treatment solution comprising a formulation for machine dishwashing as defined in claim 14.

## Revendications

1. Utilisation d'une composition polymère sous forme de gel, pouvant être obtenue par un procédé selon lequel
a) une composition de monomères M) est préparée, qui est constituée par
A) au moins un acide α-β-éthyléniquement insaturé et qui ne contient pas de monomères à effet réticulant B), qui comprennent deux ou plus de deux doubles liaisons α,β-éthyléniquement insaturées polymérisables par molécule,
b) la composition de monomères M) préparée à l'étape a) est soumise à une polymérisation radicalaire en présence d'au moins un composant polyéther PE), qui est choisi parmi les polyétherols ayant un poids moléculaire moyen en nombre d'au moins 200 g/mol et leurs mono- et di-(éthers alkyliques en C₁-C₆), les tensioactifs contenant des groupes polyéther et leurs mélanges,
dans des formulations pour le lavage de la vaisselle en machine.

2. Utilisation selon la revendication 1, dans laquelle la polymérisation radicalaire à l'étape b) a en outre lieu en présence d'un solvant LM), qui est choisi parmi l'eau, les alcanols en C₁-C₆, les polyols différents de PE), leurs éthers mono- et dialkyliques et leurs mélanges.

3. Utilisation selon l'une quelconque des revendications précédentes, dans laquelle la polymérisation à l'étape b) a lieu en mode d'alimentation, au moins une partie du composant polyéther PE) et éventuellement, s'il est présent, au moins une partie du solvant LM) étant chargées initialement, et au moins une partie de la composition de monomères préparée à l'étape a) et au moins un démarreur radicalaire S) étant introduits dans la préparation.

4. Utilisation selon l'une quelconque des revendications 2 ou 3, dans laquelle la polymérisation radicalaire à l'étape b) a en outre lieu en présence d'un solvant LM), et
- le mélange réactionnel contient au début de la réaction à l'étape b) le solvant LM) en une quantité de 0 à 90 % en poids, de préférence de 0,1 à 70 % en poids, par rapport au poids total du mélange réactionnel,
- le mélange réactionnel contient après la fin de la réaction à l'étape b) le solvant LM) en une quantité de 0 à 50 % en poids, de préférence de 0,1 à 40 % en poids, par rapport au poids total du mélange réactionnel.

5. Utilisation selon l'une quelconque des revendications précédentes, dans laquelle la composition de monomères préparée à l'étape a) est constituée par de l'acide acrylique A1) et éventuellement au moins un autre acide α,β-éthyléniquement insaturé A2), qui est choisi parmi les acides carboxyliques, les acides sulfoniques, les acides phosphoniques et leurs mélanges.

6. Utilisation selon l'une quelconque des revendications 1 à 5, dans laquelle la composition de monomères M) est exclusivement constituée par de l'acide acrylique.

7. Utilisation selon l'une quelconque des revendications précédentes, dans laquelle le composant polyéther PE) comprend au moins un polyétherol ayant un poids moléculaire moyen en nombre dans la plage allant d'environ 200 à 100 000 ou un mono ou di-(éther alkylique en C₁-C₂) de celui-ci.

8. Utilisation selon l'une quelconque des revendications précédentes, dans laquelle le composant polyéther PE) comprend au moins un tensioactif contenant des groupes polyéther, choisi parmi les éthers alkyliques de polyoxyalkylène, les éthers aryliques de polyoxyalkylène, les éthers alkylaryliques de polyoxyalkylène, les graisses et/ou les huiles animales et/ou végétales alcoxylées, les alcoxylates d'amines grasses, les alcoxylates d'amides d'acides gras, les alcoxylates de diéthanolamides d'acides gras, les esters de polyoxyéthylène-sorbitane et d'acides gras, les alkylpolyéther-sulfates, les arylpolyéther-sulfates, les alkylarylpolyéther-sulfates, les alkylpolyéther-sulfonates, les arylpolyéther-sulfonates, les alkylarylpolyéther-sulfonates, les alkylpolyéther-phosphates, les arylpolyéther-phosphates, les alkylarylpolyéther-phosphates, les éther-sulfonates de glycérine, éther-sulfates de glycérine, les (éther)sulfates de monoglycéride, les éther-sulfates d'amides d'acides gras, les esters de polyoxyalkylène-sorbitane et d'acides gras et leurs mélanges.

9. Utilisation selon l'une quelconque des revendications précédentes, dans laquelle la composition polymère présente une teneur en groupes acides de 1,5 mmol/g à 15 mmol/g, de préférence de 4 mmol/g à 7 mmol/g.

10. Utilisation selon l'une quelconque des revendications précédentes, dans laquelle la composition polymère présente une transparence, indiquée en tant que valeur T_{L}, mesurée à 500 nm, d'au moins 85 %, de manière particulièrement préférée d'au moins 90 %, par rapport à la transparence de l'eau.

11. Utilisation selon l'une quelconque des revendications 1 à 10, dans laquelle jusqu'à 80 % en moles des groupes acides, de préférence jusqu'à 70 % en moles des groupes acides, notamment jusqu'à 20 % en moles des groupes acides, se présentent sous forme neutralisée dans la composition polymère.

12. Utilisation selon l'une quelconque des revendications 1 à 10, dans laquelle les groupes acides se présentent sous forme non neutralisée dans la composition polymère.

13. Utilisation selon l'une quelconque des revendications précédentes, dans laquelle les polymères contenus dans la composition polymère présentent un poids moléculaire moyen en poids, déterminé par chromatographie par perméation de gel (CPG) en utilisant un acide polyacrylique neutralisé en tant qu'étalon polymère, de 1 000 à 70 000 Dalton.

14. Utilisation selon l'une quelconque des revendications précédentes, dans laquelle la formulation pour le lavage de la vaisselle en machine comprend les composants suivants :
a) au moins une composition polymère telle que définie dans l'une quelconque des revendications 1 à 13,
b) au moins un adjuvant,
c) éventuellement au moins une enzyme,
d) éventuellement au moins un agent blanchissant,
e) de l'eau,
f) éventuellement au moins un épaississant, et
g) éventuellement au moins un additif supplémentaire, qui est de préférence choisi parmi les tensioactifs différents de a), les bases, les inhibiteurs de corrosion, les antimousses, les colorants, les parfums, les charges, les solubilisants et les solvants organiques.

15. Procédé de lavage de la vaisselle en machine, selon lequel la vaisselle à laver est mise en contact avec une solution de traitement qui contient une formulation pour le lavage de la vaisselle en machine telle que définie dans la revendication 14.
